# EUROPEAN PATENT SPECIFICATION

(11) **EP 2 736 325 B1**
(45) Date of publication and mention of the grant of the patent: **10.10.2018**
(21) Application number: 12748281.8
(22) Date of filing: 26.07.2012
(51) Int. Cl.: A01K 67/027, C12N 9/12

(54) **PIK3CA H1047R KNOCK-IN NON-HUMAN ANIMAL BREAST CANCER MODEL**
NICHT-MENSCHLICHES PIK3CA-H1047R-KNOCK-IN-BRUSTKREBSMODELL
MODÈLE DE CANCER DU SEIN D'ANIMAL NON HUMAIN KNOCK-IN PIK3CA H1047R

(30) Priority: 28.07.2011 US 201161574161 P
(43) Date of publication of application: 04.06.2014
(73) Proprietor: F. Hoffmann-La Roche AG, 4070 Basel (CH)
(72) Inventor: SESHAGIRI, Somasekar, South San Francisco, CA 94080 (US)
(74) Representative: Townsend, Carolin
(86) International application number: PCT/US2012/000336
(87) International publication number: WO 2013/015833

(56) References cited:
- WO-A1-2011/060380
- WO-A2-2005/091849
- US-A1- 2011 060 605
- ADAMS JESSICA R ET AL: "Cooperation between Pik3ca and p53 Mutations in Mouse Mammary Tumor Formation", CANCER RESEARCH, vol. 71, no. 7, April 2011 (2011-04), pages 2706-2717, XP002687640, cited in the application
- MEYER DOMINIQUE S ET AL: "Luminal Expression of PIK3CA Mutant H1047R in the Mammary Gland Induces Heterogeneous Tumors", CANCER RESEARCH, vol. 71, no. 13, 1 April 2011 (2011-04-01) , pages 4344-4351, XP002687641, cited in the application
- W YUAN ET AL: "Conditional activation of Pik3caH1047R in a knock-in mouse model promotes mammary tumorigenesis and emergence of mutations", ONCOGENE, 1 January 2012 (2012-01-01), XP055044572, ISSN: 0950-9232, DOI: 10.1038/onc.2012.53 cited in the application
- KINROSS KATHRYN M ET AL: "An activating Pik3ca mutation coupled with Pten loss is sufficient to initiate ovarian tumorigenesis in mice.", THE JOURNAL OF CLINICAL INVESTIGATION 1 FEB 2012, vol. 122, no. 2, 1 February 2012 (2012-02-01), pages 553-557, XP002687642, ISSN: 1558-8238
- TIKOO ANJALI ET AL: "Physiological levels of Pik3ca(H1047R) mutation in the mouse mammary gland results in ductal hyperplasia and formation of ER[alpha]-positive tumors.", PLOS ONE 2012, vol. 7, no. 5, 2012, page e36924, XP002687643, ISSN: 1932-6203
- POLITI KATERINA ET AL: "How genetically engineered mouse tumor models provide insights into human cancers.", JOURNAL OF CLINICAL ONCOLOGY : OFFICIAL JOURNAL OF THE AMERICAN SOCIETY OF CLINICAL ONCOLOGY 1 JUN 2011, vol. 29, no. 16, 1 June 2011 (2011-06-01), pages 2273-2281, XP002687644, ISSN: 1527-7755
- DI NICOLANTONIO FEDERICA ET AL: "Replacement of normal with mutant alleles in the genome of normal human cells unveils mutation-specific drug responses", PROCEEDINGS OF THE NATIONAL ACADEMY OF SCIENCES, NATIONAL ACADEMY OF SCIENCES, US, vol. 105, no. 52, 30 December 2008 (2008-12-30), pages 20864-20869, XP002515482, ISSN: 0027-8424, DOI: 10.1073/PNAS.0808757105
- PANG HUAN ET AL: "Differential Enhancement of Breast Cancer Cell Motility and Metastasis by Helical and Kinase Domain Mutations of Class IA Phosphoinositide 3-Kinase", CANCER RESEARCH, vol. 69, no. 23, December 2009 (2009-12), pages 8868-8876, XP002687645, ISSN: 0008-5472
- GUPTA SURBHI ET AL: "Binding of Ras to phosphoinositide 3-kinase p110 alpha is required for Ras-driven tumorigenesis in mice", CELL, vol. 129, no. 5, June 2007 (2007-06), pages 957-968, XP002687646, ISSN: 0092-8674
- ISAKOFF STEVEN J ET AL: "Breast cancer-associated PIK3CA mutations are oncogenic in mammary epithelial cells", CANCER RESEARCH, AMERICAN ASSOCIATION FOR CANCER RESEARCH, US, vol. 65, no. 23, 1 December 2005 (2005-12-01), pages 10992-11000, XP002534440, ISSN: 0008-5472, DOI: 10.1158/0008-5472.CAN-05-2612 cited in the application
- BADER ANDREAS G ET AL: "Cancer-specific mutations in PIK3CA are oncogenic in vivo.", PROCEEDINGS OF THE NATIONAL ACADEMY OF SCIENCES OF THE UNITED STATES OF AMERICA 31 JAN 2006, vol. 103, no. 5, 31 January 2006 (2006-01-31), pages 1475-1479, XP002687647, ISSN: 0027-8424 cited in the application
- ZHAO LI ET AL: "Helical domain and kinase domain mutations in p110 alpha of phosphatidylinositol 3-kinase induce gain of function by different mechanisms", PROCEEDINGS OF THE NATIONAL ACADEMY OF SCIENCES OF THE UNITED STATES OF AMERICA, vol. 105, no. 7, February 2008 (2008-02), pages 2652-2657, XP002687648, ISSN: 0027-8424 cited in the application
- SAMUELS Y ET AL: "High frequency of mutations of the PIK3CA gene in human cancers", SCIENCE, AMERICAN ASSOCIATION FOR THE ADVANCEMENT OF SCIENCE, WASHINGTON, DC; US, vol. 304, no. 5670, 23 April 2004 (2004-04-23), page 554, XP002375241, ISSN: 0036-8075, DOI: 10.1126/SCIENCE.1096502 cited in the application & Samuels et al.: "High frequency of mutations of the PIK3CA gene in human cancers, Supporting Online Material", , vol. 304, no. 5670 23 April 2004 (2004-04-23), Retrieved from the Internet: URL:http://www.sciencemag.org/content/supp l/2004/04/27/1096502.DC1/Samuels.SOM.pdf [retrieved on 2013-07-05]
- SAMUELS Y ET AL: "Oncogenic mutations of PIK3CA in human cancers", CELL CYCLE, LANDES BIOSCIENCE, US, vol. 3, no. 10, 1 October 2004 (2004-10-01), pages C17-C20, XP008110710, ISSN: 1538-4101
- BIJAY S. JAISWAL ET AL: "Combined Targeting of BRAF and CRAF or BRAF and PI3K Effector Pathways Is Required for Efficacy in NRAS Mutant Tumors", PLOS ONE, vol. 4, no. 5, 27 May 2009 (2009-05-27), page e5717, XP055063381, DOI: 10.1371/journal.pone.0005717
- JING ZHANG ET AL: "Targeting PI3K Signaling as a Therapeutic Approach for Colorectal Cancer", GASTROENTEROLOGY, ELSEVIER, PHILADELPHIA, PA, vol. 141, no. 1, 16 May 2011 (2011-05-16), pages 50-61, XP028096615, ISSN: 0016-5085, DOI: 10.1053/J.GASTRO.2011.05.010 [retrieved on 2011-05-17]

## Description

### FIELD OF THE INVENTION

The present invention concerns the development of a *PIK3CA* H1047R knock-in non-human animal breast cancer model, and its use for identification of a spontaneous loss-of-function TP53 mutation involved in spindle cell tumor formation. The invention further concerns the identification of additional somatic mutations and copy number aberrations in the breast tumors using this model, and methods and means for the diagnosis and treatment of breast cancer.

### BACKGROUND OF THE INVENTION

The class IA PI3K catalytic subunits, p110α, p110β and p110δ, occur as an obligate heterodimer in complex with p85α, p55α, p50α, p85β or p55γ regulatory subunits In normal cells, PI3Ks are maintained in a basal inactive state and become active following growth factor stimulation and cell surface receptor engagement. Activated PI3Ks phosphorylate and convert phosphatidylinositol 4, 5 bisphosphate (PIP2) leading to phosphatidylinositol 3, 4, 5 trisphosphate (PIP3). Elevated cellular PIP3 levels promote activation of PI3K-effectors which in-turn regulate a number of cellular processes, including cell growth, proliferation and survival. (See, Cantley, L.C. Science 296, 1655-7 (2002); Hawkins, P.T., et al., Biochem Soc Trans 34, 647-62 (2006); and Vanhaesebroeck, B., et al., Trends Biochem Sci 30, 194-204 (2005)).

Frequent somatic mutations in *PIK3CA,* the gene that encodes p110α, have been reported in colorectal, breast and liver cancers (Samuels, Y. et al. Science 304, 554 (2004); Bachman, K.E. et al. Cancer Biol Ther 3, 772-5 (2004); Lee, J.W. et al. Oncogene 24, 1477-1480 (2004)). Majority of the reported p110α mutations occur in three hotspots - two (E542K and E545K) in the helical domain and one (H1047R) in the kinase domain (Bader, A.G., et al., Nat Rev Cancer 5, 921-9 (2005); Zhao, L. & Vogt, P.K. Proc Natl Acad Sci USA 105, 2652-7 (2008)). These hotspot mutations result in an oncogenic p110α capable of constitutively activating downstream signaling (Zhao, L. & Vogt, supra). Further, the p110α mutants when expressed in cells can lead to cellular transformation and support tumor formation in xenograft models (Samuels et al., supra; Zhao, L. & Vogt, Oncogene 27, 5486-5496 (2008); Isakoff, S.J. Cancer Research 65, 10992-11000 (2005); Bader, A.G. Proceedings of the National Academy of Sciences 103, 1475-1479 (2006); Horn, S. et al. Oncogene 27, 4096-4106 (2008)).

Genetically engineered mouse models (GEMMs) of human cancer serve as an important resource for understanding tumor initiation and progression (Walrath, J.C., Hawes, J.J., Van Dyke, T. & Reilly, K.M. Genetically engineered mouse models in cancer research. Adv Cancer Res 106, 113-64 (2010)). The GEMMs have also been used to test therapeutics and develop treatment strategies (Singh, M. et al. Assessing therapeutic responses in Kras mutant cancers using genetically engineered mouse models. Nat Biotechnol 28, 585-93 (2010)). Studies aimed at understanding oncogenic transformation by mutant *PIK3CA* have primarily used cell based systems, xenograft models and transgenic models (Samuels et al. supra; Isakoff, supra; Bader, A.G., supra; Horn, S. *et al.,* supra; Engelman, J.A. et al. Nat Med 14, 1351-6 (2008); Adams, J.R. et al. Cancer Research 71, 2706-2717 (2011)' Meyer, D.S. et al. Cancer Research (2011)). Given the high frequency of *PIK3CA* mutations and on-going efforts to develop small molecule inhibitors, a knock-in mouse model that can express endogenous levels of mutant *PIK3CA* from its native promoter and genomic architecture would be a valuable tool to study tumor initiation, progression and treatment. Also, such a model would lend itself to identification of lesions that cooperate with mutant PIK3CA during tumor development.

With this in mind we generated a genetically engineered mouse model where we have modified the endogenous *PIK3CA* allele by placing a dormant copy of an oncogenic mutant *PIK3CA* exon 20, encoding H1047R, adjacent to the wild-type coding exon 20. Prior to activation of the dormant mutant allele, the engineered mice express the modified wild-type *PIK3CA* (*PIK3CA^{e20mwt}*)*.* However, upon Cre-mediated recombination the mutant *PIK3CA* H1047R allele, *PIK3CA^{e20H1047R},* is activated leading to its expression. We show that the conditional activation of *PIK3CA* H1047R allele in mouse mammary epithelium leads to mutant *PIK3CA* H1047R expression and tumorigenesis.

Though next generation sequencing has enabled base pair level characterization of human tumors as they evolve and progress (Shah, S.P. et al. Nature 461, 809-813 (2009); Ding, L. et al. Nature 464, 999-1005 (2010)), mouse models of cancer provide a defined experimentally tractable system that allows for systematic sampling and characterization of tumors as they evolve.

In an effort to characterize the tumors at sequence level, we performed a whole exome capture and sequencing of tumors and identified the emergence of a spontaneous loss-of-function TP53 mutation as a potential cooperating event involved in spindle cell tumor formation. The present invention further concerns the identification of additional somatic mutations and copy number aberrations in the breast tumors from this model. In addition to molecular characterization, the ability of a PI3K small molecule inhibitor has been tested for efficacy and the result show that the tumors respond to inhibitor treatment.

### SUMMARY OF THE INVENTION

In all embodiments, the non-human transgenic mammal is a rodent, such as a mouse or a rat. In one aspect, the invention concerns a non-human transgenic mammal comprising a modified endogenous *PIK3CA* locus, wherein said modified endogenous *PIK3CA* locus comprises a dormant mutant *PIK3CA* exon 20 allele encoding a H1047R mutation adjacent to wild-type exon 20 followed by a transcriptional stop cassette, wherein said wild-type exon 20 followed by a transcriptional stop cassette is flanked by loxP sites, and wherein said non-human transgenic animal is capable of conditional expression of the *PIK3CA* H1047R (*PIK3CA^{e20H1047R}*) mutant allele.

In one embodiment, the mutant allele is under control of the *PIK3CA* endogenous promoter.

In another embodiment, conditional expression is triggered by activation of the mutant allele by Cre-mediated recombination.

In yet another embodiment, conditional activation is tissue specific.

In a further embodiment, conditional activation leads to mutant PIK3CAH1047R expression and tumorigenesis.

In another aspect, the invention concerns a tumor-bearing non-human transgenic mammal conditionally expressing a mutant *PIK3CA* allele encoding a H1047R (*PIK3CA^{e20H1047R}*) mutation under control of the *PIK3CA* endogenous promoter.

In one embodiment, the mammal expresses the *PIK3CA* H1047R mutant allele in a tissue-specific manner.

In another embodiment, the *PIK3CA* H1047R mutant allele is expressed in the mammary gland of the mammal and the tumor is a mammary tumor.

In yet another embodiment, the tumor-bearing non-human transgenic mammal is heterozygous for the *PIK3CA* H1047R mutant allele.

In another embodiment, the tumor-bearing non-human transgenic mammal is homozygous for the *PIK3CA* H1047R mutant allele.

In a further embodiment, the mammary tumor is selected from the group consisting of fibroadenomas, adenocarcinomas and spindle cell neoplasia.

In a still further embodiment, the tumor-bearing non-human transgenic mammal is a rodent, such as a mouse or a rat.

In a further aspect, the invention concerns a method for making a transgenic mammal described above, comprising placing a copy of exon 20 encoding a H1047R mutation, adjacent to exon 20 encoding the wild-type *PIK3CA* allele.

In one embodiment, exon 20 encoding the wild-type *PIK3CA* allele is flanked by loxP sites, followed by a transcriptional stop cassette and a copy of *PIK3CA* exon 20 encoding the H1047R mutation.

In a further example, the invention concerns a method of screening a subject for the presence of a tumor, the method comprising testing a biological sample obtained from the subject for the presence of a *PIK3CA* H1047R allele or the corresponding gene product.

In one embodiment, the tumor is selected from the group consisting of fibroadenomas, adenocarcinomas and spindle cell neoplasias.

In another embodiment, the tumor is selected from the group consisting of breast cancer, ovarian cancer, colorectal cancer, gastric cancer, lung cancer, hepatocellular cancer, thyroid cancer, endometrial cancer, leukemia and malignancies of the central nervous system.

In yet another embodiment, the tumor is breast cancer.

In a further example, the method further comprises testing said sample for the presence of a secondary somatic mutation or copy number alteration, where the secondary somatic mutation may, for example, be in the TP53 gene, such as a R248H TP53 mutation. In other embodiments, the secondary somatic mutation may be in the Plk1, Tssk2 and/or SMG1 gene.

In another aspect, the invention concerns a method of screening a drug candidate for the treatment of tumor comprising (a) administering a drug candidate to the tumor-bearing non-human animal of claim 1, and (b) measuring the response of the tumor to said treatment.

In one embodiment, step (b) comprises evaluating the ability of the drug candidate to evoke at least one response selected from the group consisting of reduction of the number of tumor cells, reduction of tumor size or tumor load, inhibition of tumor cell infiltration into peripheral organs, inhibition of tumor metastasis, and inhibition of tumor growth.

In another embodiment, the tumor is selected from the group consisting of fibroadenomas, adenocarcinomas and spindle cell neoplasias.

In yet another embodiment, the tumor is selected from the group consisting of breast cancer, ovarian cancer, colorectal cancer, gastric cancer, lung cancer, hepatocellular cancer, thyroid cancer, endometrial cancer, leukemia and malignancies of the central nervous system.

In a further embodiment, the tumor is breast cancer.

In a different aspect, the invention concerns a method for identifying an anti-cancer agent for the treatment of drug-resistant cancer, comprising (a) administering a drug candidate to the tumor-bearing non-human animal of claim 1, (b) measuring the response of the tumor to the treatment, and (c) identifying the drug candidate as an anti-cancer agent for the treatment of drug-resistance cancer if the tumor exhibits a positive response to the treatment.

In one embodiment, the positive response is selected from the group consisting of reduction of the number of tumor cells, reduction of tumor size or tumor load, inhibition of tumor cell infiltration into peripheral organs, inhibition of tumor metastasis, and inhibition of tumor growth.

In another embodiment, the tumor is selected from the group consisting of breast cancer, ovarian cancer, colorectal cancer, gastric cancer, lung cancer, hepatocellular cancer, thyroid cancer, endometrial cancer, leukemia and malignancies of the central nervous system.

In a particular embodiment, the tumor is breast cancer.

### BRIEF DESCRIPTION OF THE DRAWINGS

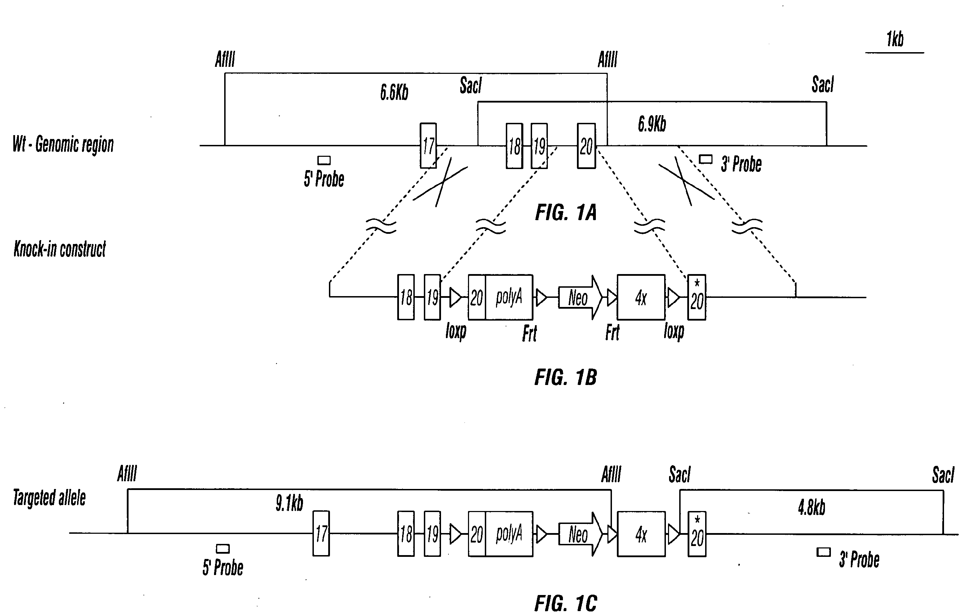

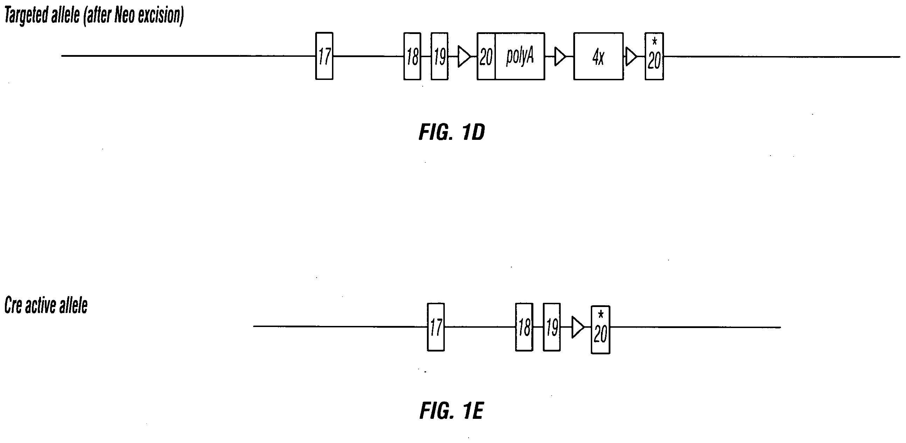

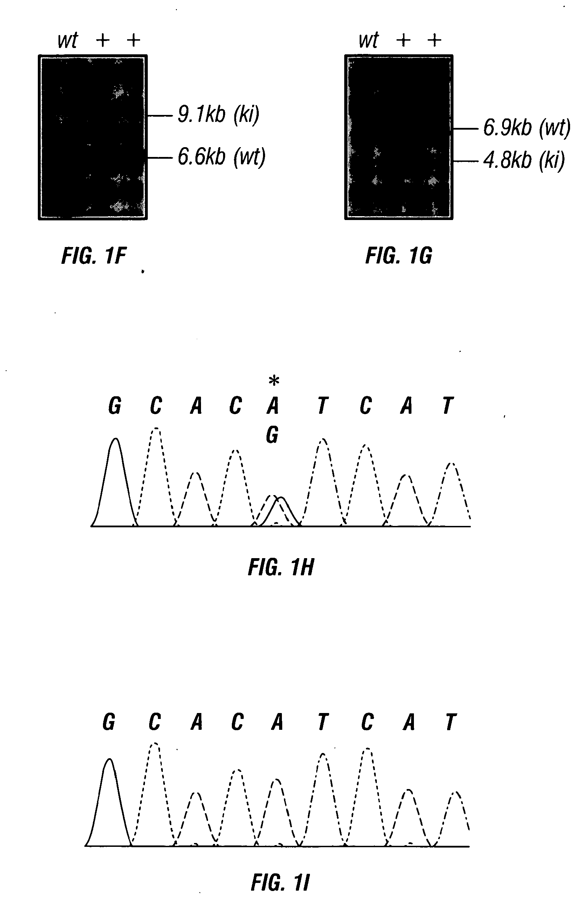

**Figure 1** Generation of *PIK3CA^{e20H1047R}* conditionally activatable knock-in allele. a-d. (a) Genomic locus encoding *PIK3CA* locus, (b) the targeting construct, (c) the targeted allele, (d) the targeted locus in the ES after removal of the neomycin cassette and (e) *PIK3CA ^{e20H1047R}* allele following Cre-mediate activation are shown. f-g. Southern blot with a 5' probe (f) and a 3' probe (g) was used to identify the appropriately targeted knock-in (ki) and wild-type (wt) allele. h-i. Sanger sequencing of the cDNA obtained from *PIK3CA*^{*e20H1047R*/+} mammary gland (h) and kidney (i) following Cre-mediated activation confirms the expression of the mutant allele in the mammary gland.

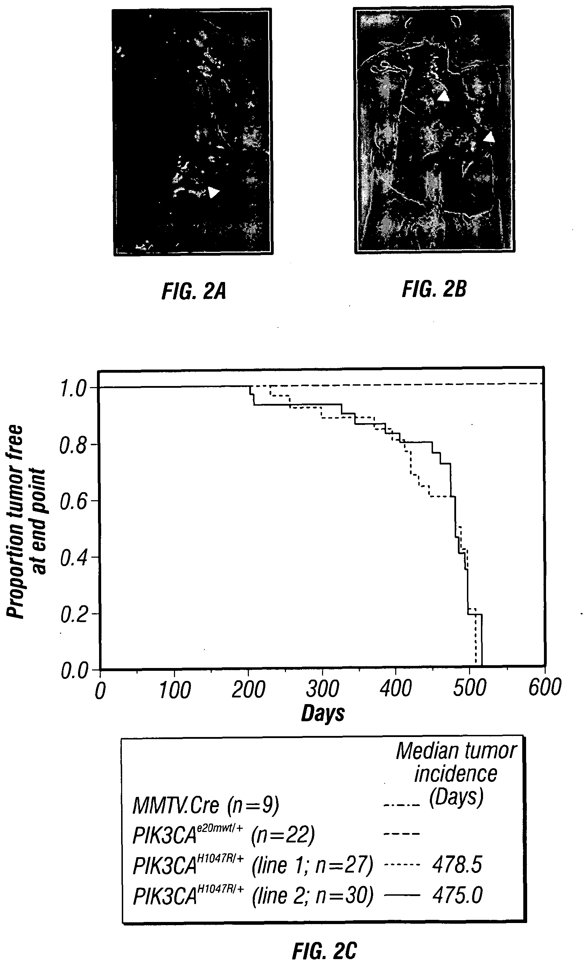

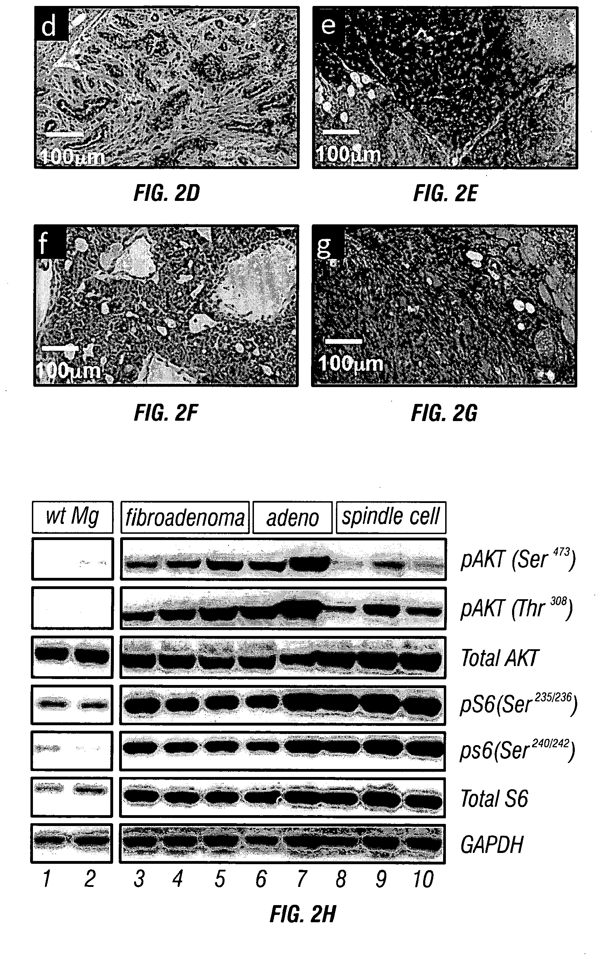

**Figure 2** *PIK3CA^{e20H1047R}* mice develop mammary tumors. a-b. Mouse bearing tumor in the abdominal mammary (a) and thoracic mammary (b) glands. White arrow heads points to the location of the tumors. c. Kaplan-Meier plot depicting tumor free survival of two independent *PIK3CA^{e20H1047R}* lines following MMTV-Cre mediated activation of the latent *PIK3CA*^{e20mwt} allele. Only animals bearing mammary tumors (-2500 mm³; 86.4% of line 1 and 84.2% of line 2 PIK3CA^{e20H1047R/+} female mice observed in the study had developed tumors) at end point are shown in the plot. Two control groups, MMTV-Cre mice and *PIK3CA*^{e20mwt}*,* were tumor free for the duration of the study. d-g. Histology of *PIK3CA^{e20H1047R}* mammary tumors. Analysis of H&E stained tumors sections show presence of multiple histological types which includes fibroadenoma (d), adenosquamous carcinoma (e), adenocarcinoma (f) and spindle cell tumor (g). h. Western blot of *PIK3CA^{e20H1047R}* mammary tumors representative of the histological types show elevated pAKT levels (lanes 3-10) and pS6 in all the tumor types compared to control mammary glands (lane 1-2). Tumors 9, 10 were derived from passaging a primary spindle tumor in SCID mice mammary fat pad.

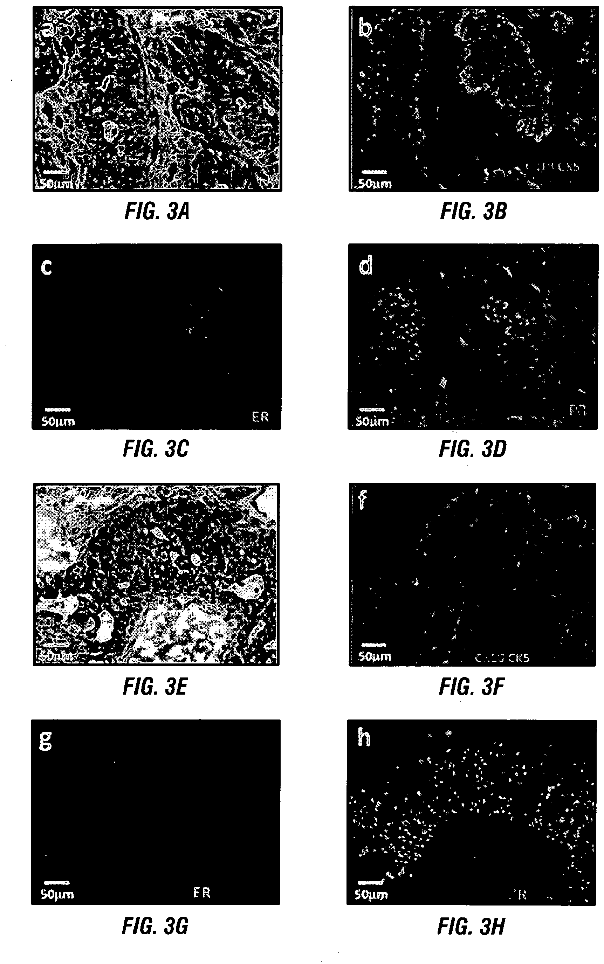

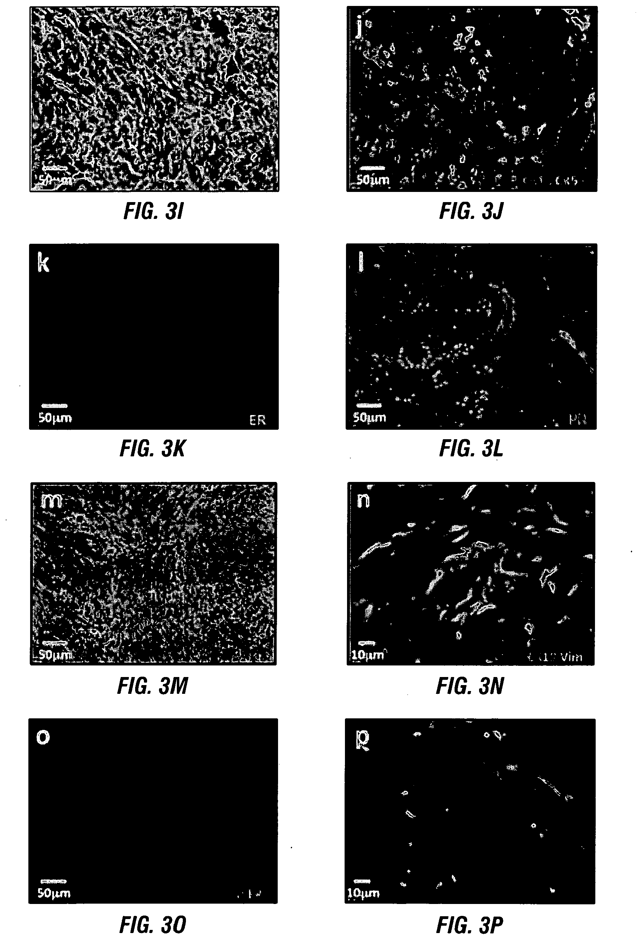

**Figure 3** Immunofluorescence staining of *PIK3CA^{e20H1047R}* mammary tumors. a-p. Serial sections of fibroadenoma (a-d), adenocarcinoma (e-h & i-1) and spindle cell tumor (m-p) stained with H&E (a, e, l & m), anti-CK18 & anti-CK5 (b, f & j), anti-ERa (c, g & k), anti-Era and anti-PR (o), anti-PR (d, h & l), and anti-CK18 & anti-vimentin (n, p) antibodies. Section in (p) was obtained from a third passage spindle cell tumor explant.

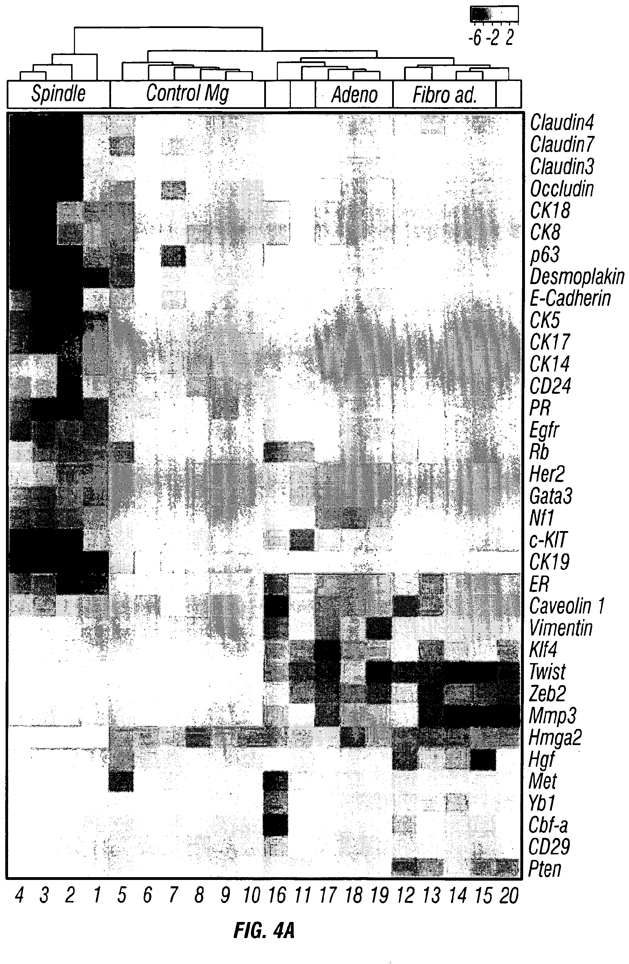

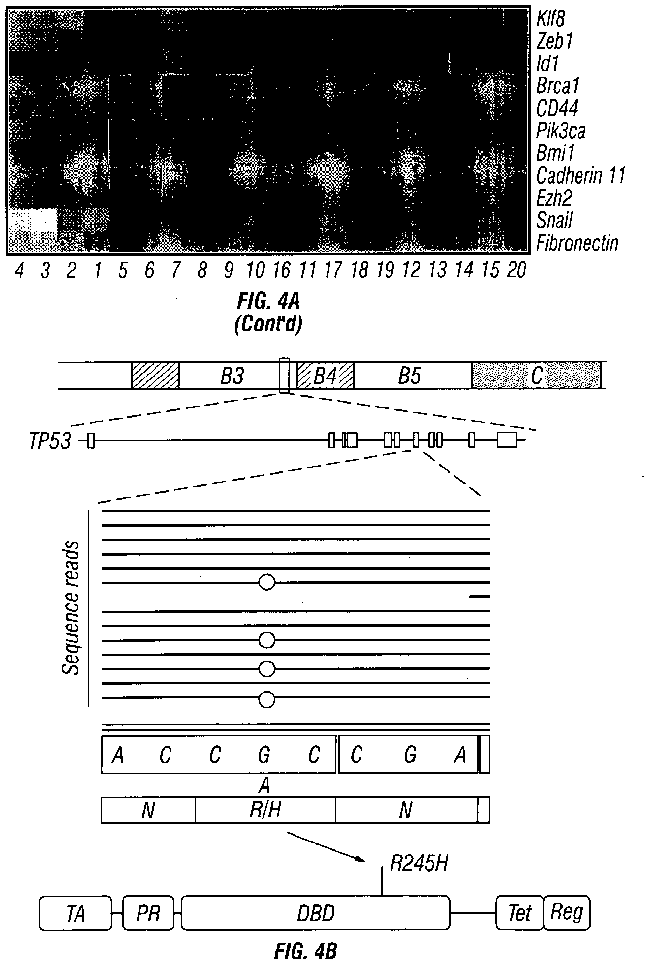

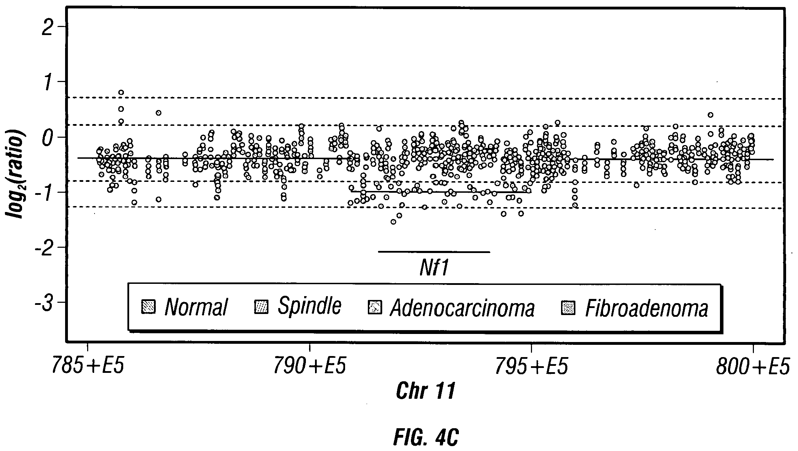

**Figure 4** Expression profile and genomic aberrations in *PIK3CA^{e20H1047R}* driven mammary tumors. **a.** Heatmap derived by hierarchical clustering of the histologically distinct mammary tumor types using expression level for the set of genes indicated. Mammary tumors of different histological types show distinct expression profiles. Samples depicted at spindle cell tumors (1-4; 2-4 are serial passages derived from 1), control mammary gland (Mg) (5-10), fibroadenoma (11-15) and adenocarinoma (16-20). Tumor samples 17 and 18 were derived by serial passage of tumor 16. Similarly, tumor sample 18 was derived from passaging tumor 11. Control mammary gland samples 6, 9 and 10 were matched samples from animals bearing tumors 12, 13 and 14, respectively. b. Whole exome sequencing identifies a spontaneous *TP53* mutation in spindle cell tumor. The genomic region, the TP53 exons (mutation shown in red), the reads from the region of interest (depicted as solid lines with mutant position show as red dot) and the mutation on the domain architecture of TP53 is shown. PR, proline-rich domain; Reg, carboxy-terminal regulatory domain; TA, transactivation domain; Tet, tetramerization domain **c.** CGH analysis reveals loss of NF-1 in spindle cell tumor.

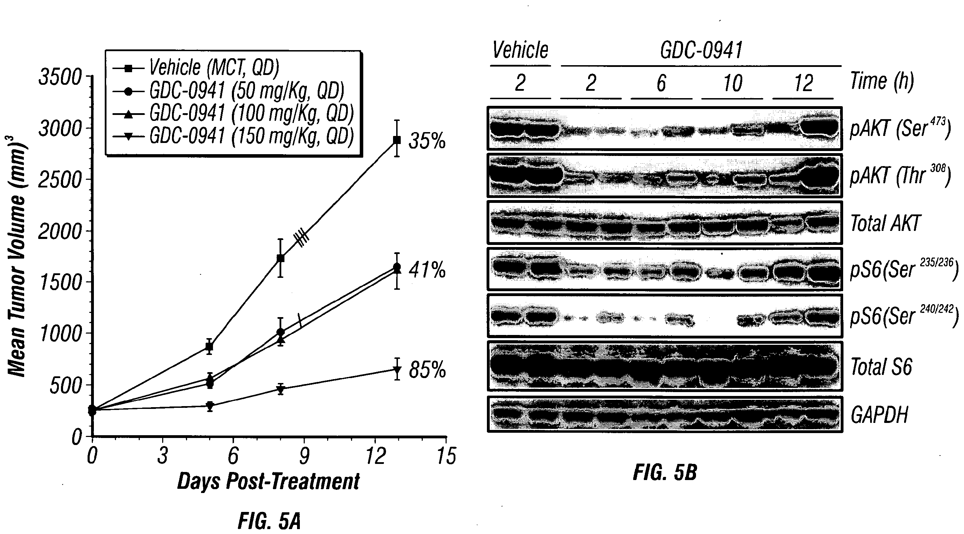

**Figure 5** *In vivo* anti-tumor activity of PI3K inhibitor. a. *PIK3CA^{20H1047R}* mammary tumor explants grown as xenografts respond to treatment with GDC-0941. Percent tumor growth inhibition with respect to control (n=9) for each of the treatment dose (n=9 for all doses) is shown on the graph. b. Tumor from animals treated with GDC-0941 show evidence of PI3K pathway inhibition.

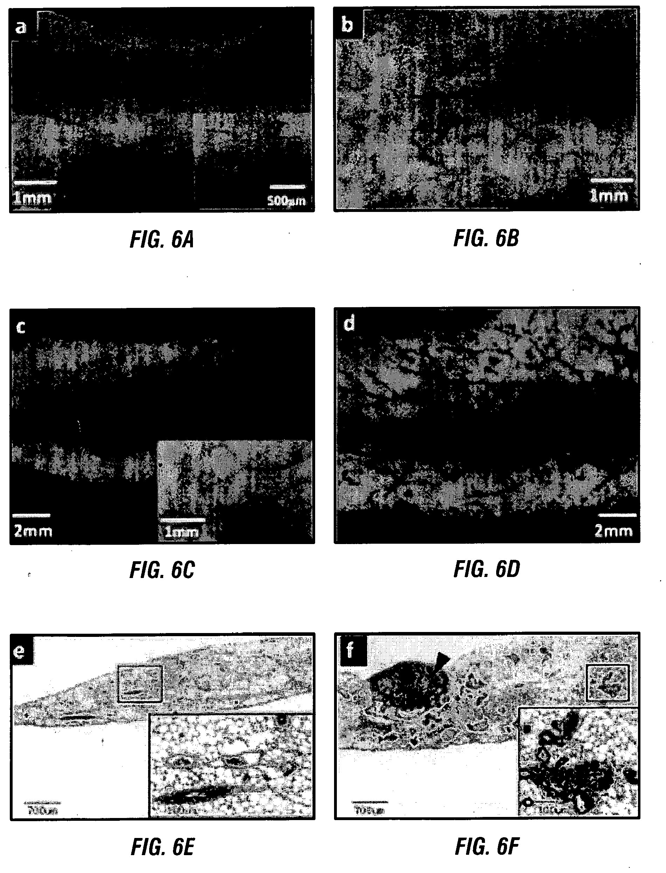

**Figure 6** *PIK3CA*^{*e20H1047R*/*+*} expressing mammary glands show excessive side branching and presence of tumor nodules. Carmine alum stained whole mount (a-d) or H&E stained sections (e, f) of abdominal mammary gland from 12 week (a, b) and 50 week (c, d, e, & f) old *MMTV-Cre* (a, c & e) or *PIK3CA*^{*e20H1047R*/+} (b, d & f) mice.

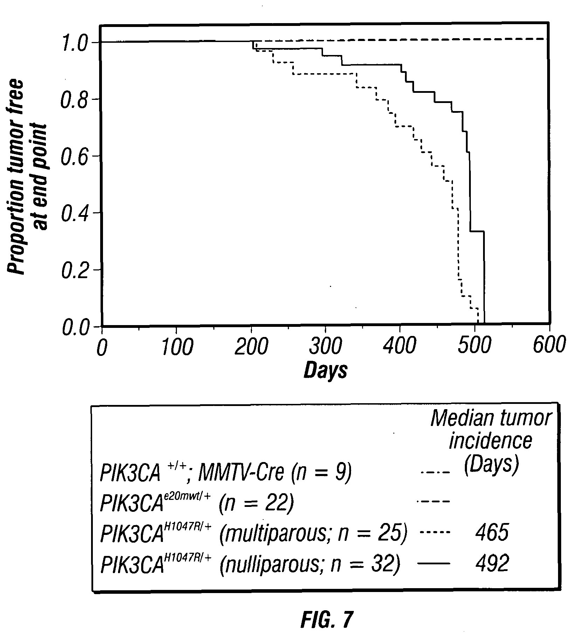

**Figure 7** Tumor free survival of multiparous and nulliparous cohorts. Kaplan-Meier analysis of median tumor free survival in multiparous (465 days) and nulliparous (492 days) is statistically different (log-rank test p-value = 0.0002). Only animals bearing mammary tumors at end point are shown in the plot

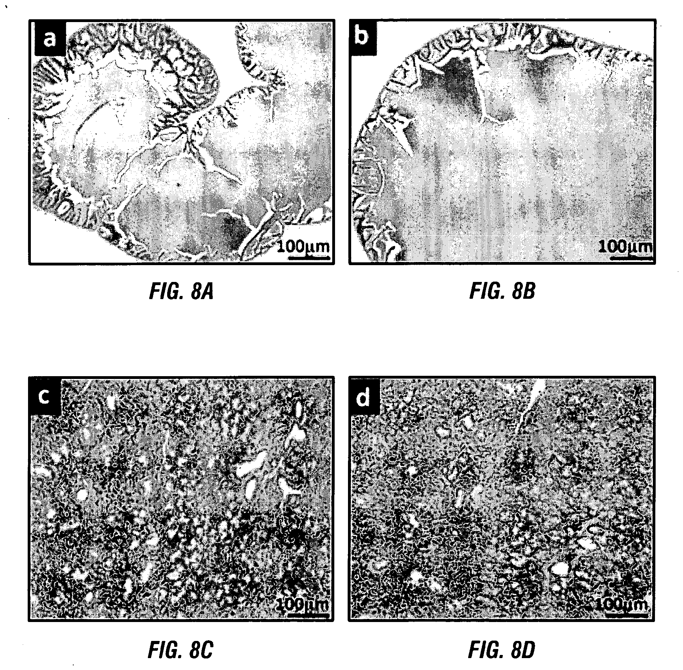

**Figure 8** Histology of seminal vesicles (a, b) and salivary glands (c, d) from *PIK3CA^{e20mwt}* (a, c) or *PIK3CA*^{*e20H1047R*/+} (b, d).

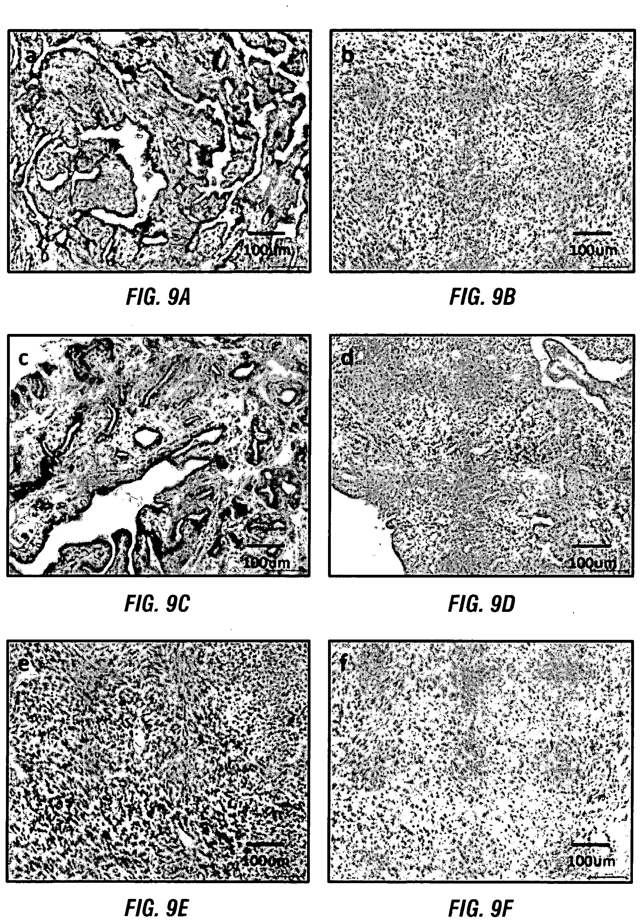

**Figure 9** Histological comparison of primary and passaged tumors. a-f. Fibroadenoma in (a) when passaged resulted in spindle cell tumor shown in (b). Similarly, fibroadenoma in (c) when passaged resulted in adenocarcinoma shown in (d). However, the spindle cell tumor (e) when passaged maintained its spindle cell histology with invasive characteristics (f).

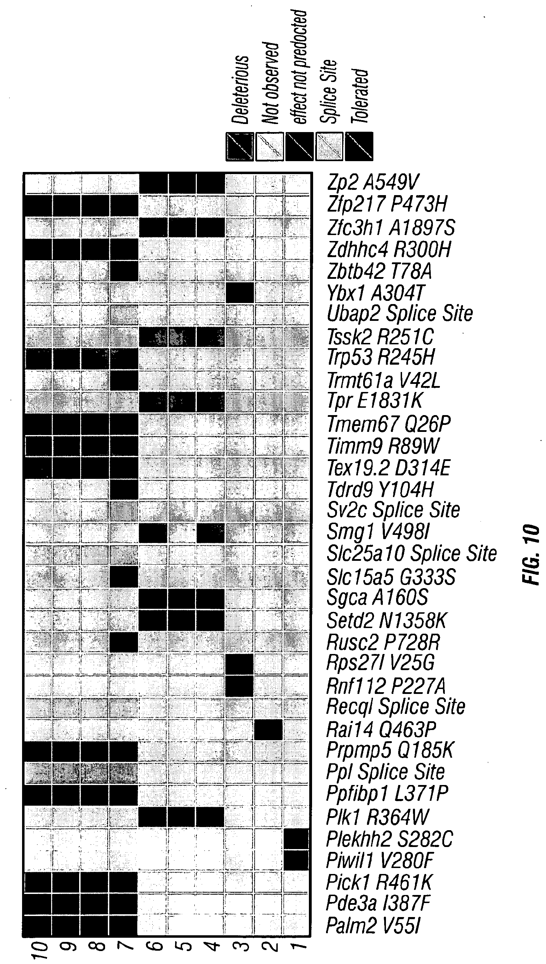

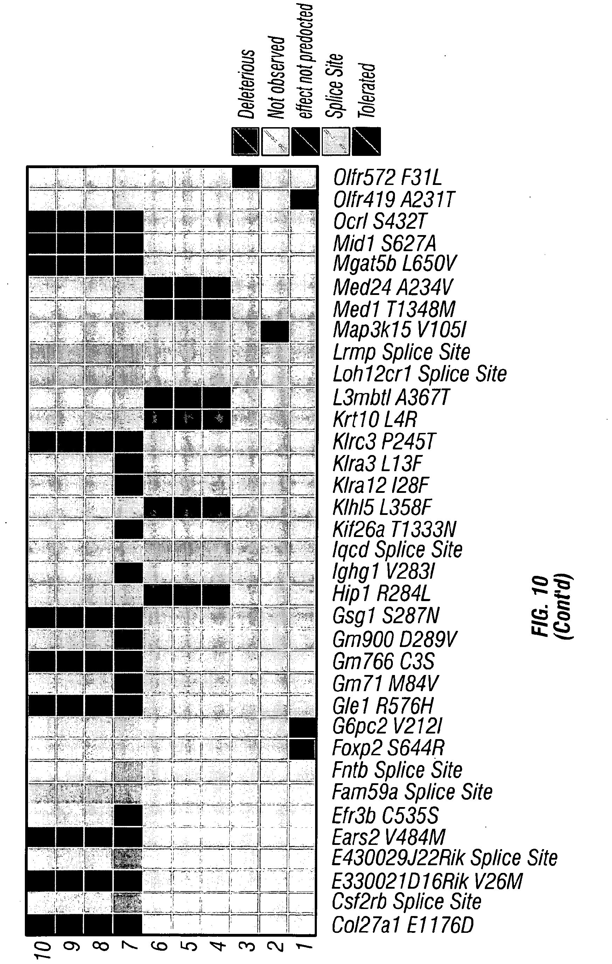

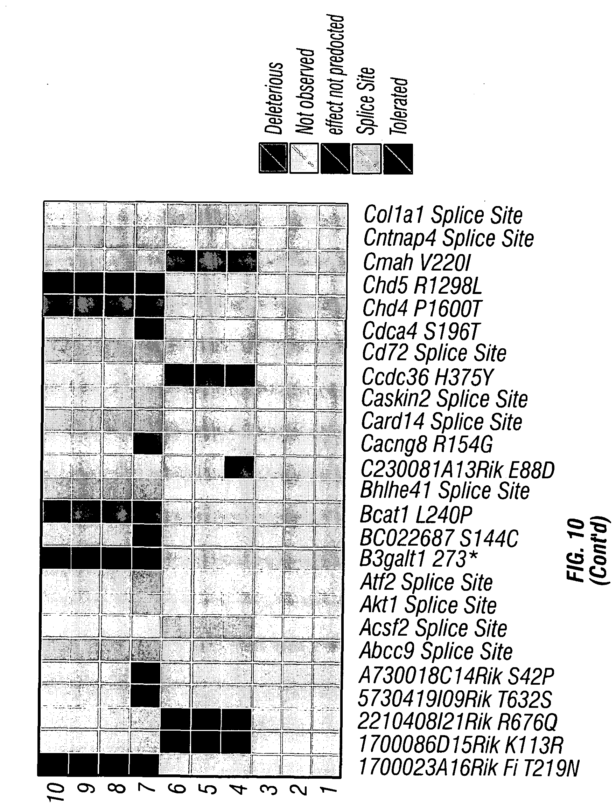

**Figure 10** A heatmap representation of the predicted somatic mutations by tumor type in the primary tumor and passaged tumors. The effect of the mutation, deleterious, tolerated, and not scored (effect not called) on gene function as predicted by SIFT (Ng, P.C. & Henikoff, S. Genome Res 12, 436-46 (2002)) is shown. 1, 2 and 3 are three different fibroadenomas, 4-6 are adenocarcinomas. 5 and 6 are tumors derived from two independent tumor 4 explants passaged in mice. 7-10 are spindle cell tumors. Tumor 7 explant was serially passaged in mice to derive tumor 8, 9 and 10.

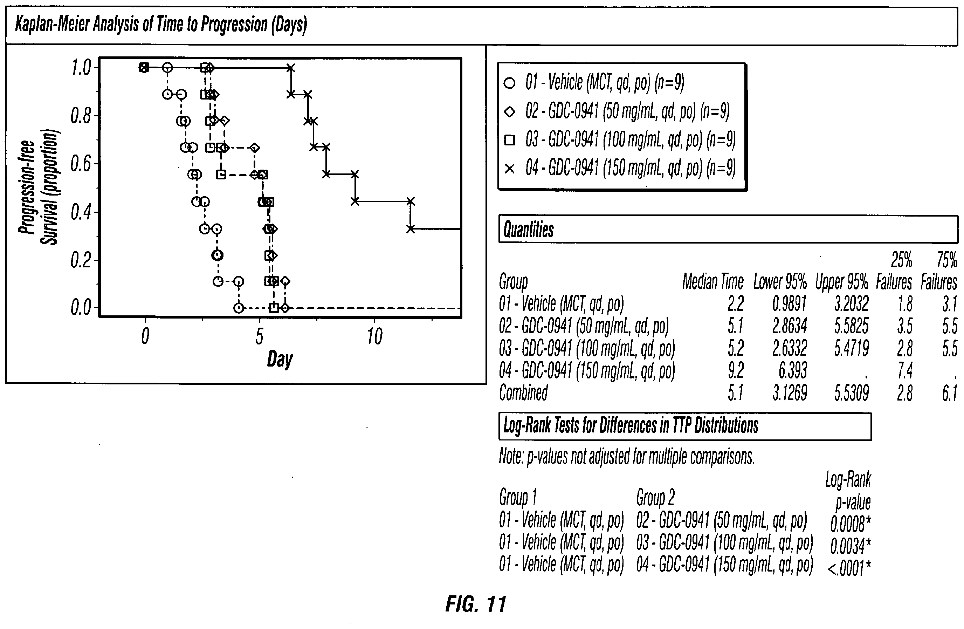

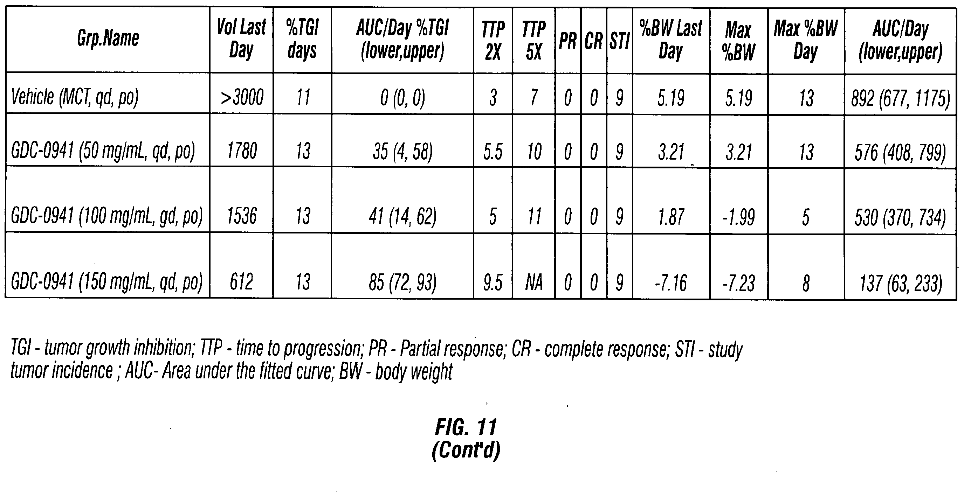

**Figure 11** Kaplan-Meier Analysis of time to progression of tumor bearing mice treated with PI3K inhibitor at the doses indicated.

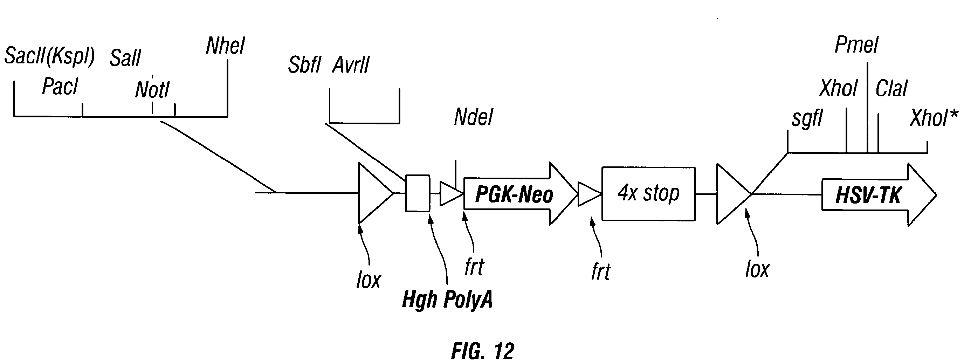

**Figure 12** Targeting template vector. The vector was engineered to provide unique restriction enzyme sites for cloning and other elements needed to generate the targeting vector.

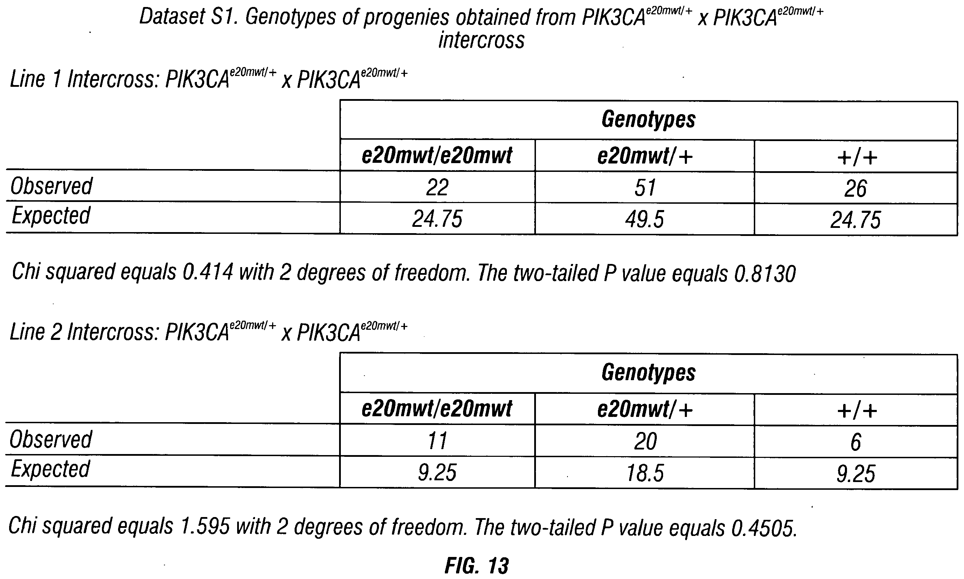

**Figure 13****.** Genotypes of progenies obtained from *PIK3CA*^{*e20mwt*/+} x *PIK3CA*^{*e20mwt*/+} intercross.

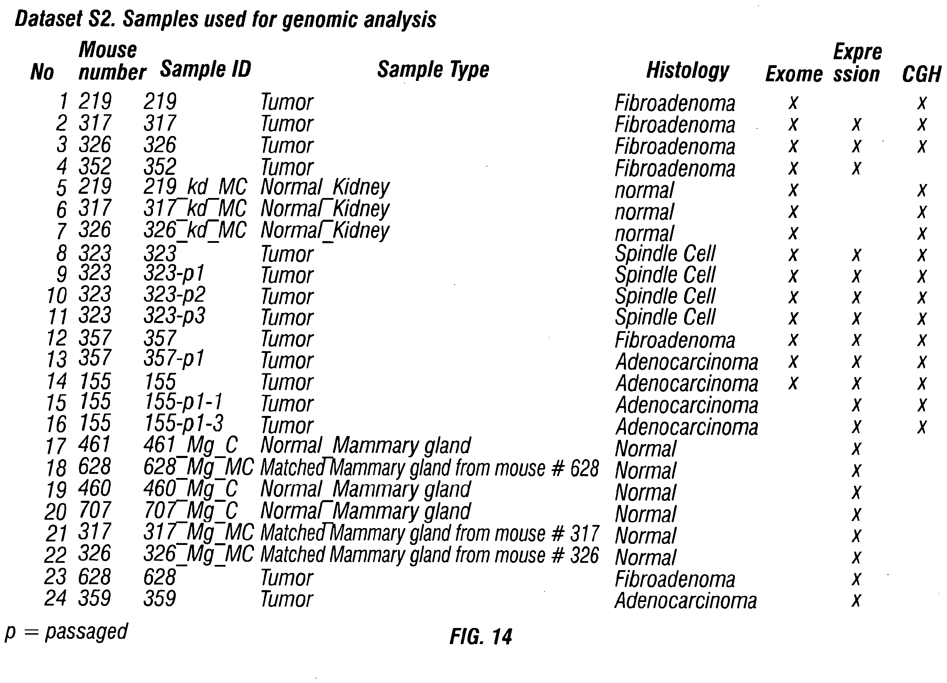

**Figure 14****.** Samples used for genomics analysis.
**Table 1.** Predicted somatic mutations in tumors.

### DETAILED DESCRIPTION OF THE INVENTION

### I. Definitions

Unless defined otherwise, technical and scientific terms used herein have the same meaning as commonly understood by one of ordinary skill in the art to which this invention belongs. Singleton et al., Dictionary of Microbiology and Molecular Biology 2nd ed., J. Wiley & Sons (New York, N.Y. 1994). One skilled in the art will recognize many methods and materials similar or equivalent to those described herein, which could be used in the practice of the present invention. Indeed, the present invention is in no way limited to the methods and materials described. For purposes of the present invention, the following terms are defined below.

As used herein, the terms "allele," "allelic variant," or "allelic variation," are used interchangeably and refer to any of two or more alternative forms of a gene occupying the same chromosomal locus. Allelic variation arises naturally through mutation, and can result in phenotypic polymorphism within populations. Gene mutations can be silent (no change in the encoded polypeptide) or can encode polypeptides having altered amino acid sequence. The term "allelic variant" also is used herein to denote a protein encoded by an allelic variant of a gene. Typically the reference form of the gene encodes a wild-type form and/or predominant form of a polypeptide from a population or single reference member of a species. Typically, allelic variants, which include variants between and among species typically have at least 80%, 90% or greater amino acid identity with a wild-type and/or predominant form from the same species; the degree of identity depends upon the gene and whether comparison is interspecies or intraspecies. Generally, intraspecies allelic variants have at least about 80%, 85%, 90% or 95% identity or greater with a wild-type and/or predominant form, including 96%, 97%, 98%, 99% or greater identity with a wild-type and/or predominant form of a polypeptide.

When a subject has two identical alleles of a gene, the subject is said to be homozygous for that gene or allele. When a subject has two different alleles of a gene, the subject is said to be heterozygous for the gene. Alleles of a specific gene can differ from each other in a single nucleotide or several nucleotides, and can include substitutions, deletions and insertions of nucleotides. An allele of a gene also can be a form of a gene containing a mutation.

As used herein, the expression "conditional expression" or "conditionally expressed" is intended to refer to transcription of a gene when a condition is met while no transcription of the gene when the condition is not met. Conditional expression can be achieved or performed naturally by the cell (i.e., without artificial intervention) or may be achieved or performed artificially (i.e., with the involvement of artificial intervention, such as for example, but not limited to, the use of regions or promoters regulated by the use of chemical agents, etc.). Conditional expression might, for example, be initiated by a recombination event triggered by a site-specific recombinase, which results in the activation of a dormant gene, such as by Cre-mediated recombination.

The term "nucleic acid" refers to polynucleotides such as deoxyribonucleic acid (DNA), and, where appropriate, ribonucleic acid (RNA). The term also includes, as equivalents, analogs of either DNA or RNA made from nucleotide analogs, and as applicable, single (sense or antisense) and double-stranded polynucleotides. An "isolated" nucleic acid molecule is a nucleic acid molecule that is identified and separated from at least one contaminant nucleic acid molecule with which it is ordinarily associated in the natural source of the nucleic acid. An isolated nucleic acid molecule is other than in the form or setting in which it is found in nature. Isolated nucleic acid molecules therefore are distinguished from the nucleic acid molecule as it exists in natural cells. However, an isolated nucleic acid molecule includes a nucleic acid molecule contained in cells that ordinarily express the encoded protein where, for example, the nucleic acid molecule is in a chromosomal location different from that of natural cells.

As used herein, the term "vector" refers to a nucleic acid molecule capable of transporting another nucleic acid to which it has been linked. The term "expression vector" includes plasmids, cosmids or phages capable of synthesizing the subject protein encoded by the respective recombinant gene carried by the vector. Preferred vectors are those capable of autonomous replication and/expression of nucleic acids to which they are linked. In the present specification, "plasmid" and "vector" are used interchangeably, as the plasmid is the most commonly used form of vector.

As used herein, the terms "transcriptional regulatory elements" and "transcriptional regulatory sequences" are used interchangeably and refer to nucleic acid, e.g. DNA sequences necessary for the expression of an operably linked coding sequence in a particular host organism. The control sequences that are suitable for prokaryotes, for example, include a promoter, optionally an operator sequence, and a ribosome binding site. Eukaryotic cells are known to utilize promoters, enhancers, splicing signals and polyadenylation signals. These terms are intended to encompass all elements that promote or regulate transcription, including promoters, core elements required for basic interaction of RNA polymerase and transcription factors, upstream elements, enhancers, and response elements (Lewin, "Genes V" (Oxford University Press, Oxford) pages 847-873). Reference herein to the transcriptional regulatory elements of a gene or class of gene includes both all or an intact region of the naturally occurring transcriptional regulatory elements and modified forms of the transcriptional regulatory elements of the gene or group of genes. Such modified forms include rearrangements of the elements, deletions of some elements or extraneous sequences, and insertion of heterologous elements. The modular nature of transcriptional regulatory elements and the absence of position-dependence of the function of some regulatory elements such as enhancers make such modifications possible. Numerous techniques are available for dissecting the regulatory elements of genes to determine their location and function. Such information can be used to direct modification of the elements, if desired. It is preferred, however, that an intact region of the transcriptional regulatory elements of a gene be used.

Nucleic acid is "operably linked" when it is placed into a functional relationship with another nucleic acid sequence. For example, DNA for a presequence or secretory leader is operably linked to DNA for a polypeptide if it is expressed as a preprotein that participates in the secretion of the polypeptide; a promoter or enhancer is operably linked to a coding sequence if it affects the transcription of the sequence; or a ribosome binding site is operably linked to a coding sequence if it is positioned so as to facilitate translation. Generally, "operably linked" means that the DNA sequences being linked are contiguous, and, in the case of a secretory leader, contiguous and in reading phase. However, enhancers do not have to be contiguous. Linking is accomplished by ligation at convenient restriction sites. If such sites do not exist, the synthetic oligonucleotide adaptors or linkers are used in accordance with conventional practice.

The term "transfection" refers to the introduction of a nucleic acid, e.g., an expression vector, into a recipient cell by nucleic acid-mediated gene transfer. "Transformation", as used herein, refers to a process in which a cell's genotype is changed as a result of the cellular uptake of exogenous DNA or RNA, and the transformed cell expresses a desired heterologous protein.

As used herein, the term "transgene" refers to a nucleic acid sequence which is partly or entirely heterologous, i.e., foreign, to the transgenic animal or cell into which it is introduced, or, is homologous to an endogenous gene of the transgenic animal or cell into which it is introduced, but which is designed to be inserted, or is inserted, into the animal's genome in such a way as to alter the genome of the cell into which it is inserted (e.g., it is inserted at a location which differs from that of the natural gene or its insertion results in a knockout). A transgene can be operably linked to one or more transcriptional regulatory sequences and any other nucleic acid, such as introns, that may be necessary for optimal expression of a selected nucleic acid.

Accordingly, the term "transgene construct" refers to a nucleic acid which includes a transgene, and (optionally) such other nucleic acid sequences as transcriptionally regulatory sequence, polyadenylation sites, replication origins, marker genes, etc., which may be useful in the general manipulation of the transgene for insertion in the genome of a host organism.

The term "transgenic" is used herein as an adjective to describe the property, for example, of an animal or a construct, of harboring a transgene. For instance, as used herein, a "transgenic organism" is any animal, preferably a non-human mammal, more preferably a rodent, in which one or more of the cells of the animal contain heterologous nucleic acid introduced by way of human intervention, such as, for example, by trangenic techniques well known in the art. The nucleic acid is introduced into the cell, directly or indirectly by introduction into a precursor of the cell, by way of deliberate genetic manipulation, such as by microinjection or by infection with a recombinant virus. The term "genetic manipulation" does not include classical cross-breeding, or in vitro fertilization, but rather is directed to the introduction of a recombinant DNA molecule. This molecule may be integrated within a chromosome, or it may be extrachromosomally replicating DNA.

A "knock-out" of a gene means an alteration in the sequence of the gene that results in a decrease of function of the target gene, preferably such that target gene expression is undetectable or insignificant. Transgenic knock-out animals can be comprise a heterozygous knock-out of a target gene, or a homozygous knock-out of a target gene. "Knock-outs" as used herein also include conditional knock-outs, where alteration of the target gene can occur upon, for example, exposure of the animal to a substance that promotes target gene alteration, introduction of an enzyme that promotes recombination at the target gene site (e.g., Cre in the Cre-lox system), or other method for directing the target gene alteration postnatally.

A "knock-in" refers to the targeted insertion of a transgene in a host cell genome that results in expression of the transgene and/or in altered expression (e.g., increased (including ectopic) or decreased expression) of an endogenous target gene, e.g., by introduction of an additional copy of the target gene, or by operatively inserting a regulatory sequence that provides for enhanced expression of an endogenous copy of the target gene. "Knock-in" transgenics can comprise a heterozygous knock-in of a transgene or a homozygous knock-in of a transgene. "Knock-ins" also encompass conditional knock-ins, where expression of a transgene and/or altered expression of an endogenous target gene can occur, for example, by exposing the animal to a substance that promotes such expression, by introducing an enzyme that promotes recombination at the site of targeted insertion (e.g., Cre in the Cre-lox system), or by some other method for altering the site of targeted insertion.

"Homozygous" state means a genetic condition existing when the same alleles reside at corresponding loci on homologous chromosomes. In contrast, "heterozygous" state means a genetic condition existing when different alleles reside at corresponding loci on homologous chromosomes.

"Site specific recombinases" are enzymes that are present in some viruses and bacteria and have been characterized to have both endonuclease and ligase properties. Site specific recombinases catalyze at least the following four events (a) deletion of a DNA fragment flanked by "compatible site-specific recombinase targeting sites" (SSRTS) in the same orientation (e.g. head-to-tail or tail-to-head); (b) inversion of a DNA fragment flanked by compatible SSRTS in opposite orientation (e.g. head-to-head or tail-to-tail); (c) integration of a cyclic DNA fragment containing a SSRTS into a compatible SSRTS; and (d) chromosomal translocation between compatible SSRTS located on different chromosomes. To perform those reactions, the site-specific recombinase has typically at least the following four activities: (1) recognition of one or two specific DNA sequences; (2) cleavage of said DNA sequence or sequences; (3) DNA topoisomerase activity involved in strand exchange; and (4) DNA ligase activity to reseal the cleaved strands of DNA (Sauer, B., Cur. Opin. Biotech. 5: 521-527, 1994).

The term "recombinase" or "site-specific recombinase" refers to enzyme(s) that carry out site specific recombination (SSR) to alter the DNA structure. This definition includes transposases, lambda integration/excision enzymes, and site-specific recombinases. Well-known examples of recombinases include, without limitation, Cre-lox, FLP/FRT, R/RS, Gin/gix, a pSR1 system, a cer system, and a fim system (e.g. N. L. Craig, Annu. Rev. Genet. 22:17, 1988); Odell et al., Homologous Recomb. Gene Silencing Plants, 1994, pp. 219-270, Paszkowski, Jerzy, ed. Kluwer: Dordrecht, Germany). Additionally, SSR systems have been identified in microorganisms such as phage, bacterium (e.g., *E. coli*), yeast and the like. This includes the E. *coli* lambda att P system for integration and excision (Zubko et al. Nature Biotechnology 18:442, 2000) and the Streptomyces phage C31 integrase (Groth et al. Proc. Natl. Acad. Sci. USA 97:5995, 2000). When the SSR system from these microorganisms is introduced into organisms (including plants) different from the organism from which this system had been derived, it behaves in the same way as in the original organism.

"Recombinase site" or "site-specific recombination sequence" means a DNA sequence that a recombinase will recognize in facilitating a recombination event. It will be appreciated that this may be a wild-type or mutant recombinase site, as long as functionality is maintained and the recombinase enzyme may still recognize the site, bind to the DNA sequence, and catalyze the recombination between two adjacent recombinase sites.

The term "floxed" refers to the flanking of a genetic element, or a portion thereof, with tandem site-specific sequences. The site-specific sequences may be oriented in the same orientation, i.e., directly repeated (such that the DNA element is removed upon SSR), or in opposite orientations from one another (such that the DNA element is inverted upon SSR). The floxed element may be a single promoter, a transcriptional 'STOP' blocking element, a promoter operably linked to a target sequence, or a combination of these and other genetic elements.

A "site-specific recombination substrate" or "SSR substrate" refers to any DNA that is a substrate of SSR, resulting from the action of the site-specific recombinase on recombinase sites. The term includes floxed DNA elements, i.e., those DNA elements flanked by recombinase sites.

The terms "founder line" and "founder animal" refer to those animals that are the mature product of the embryos to which a transgene was added, i.e., those animals that grew from the embryos into which DNA was inserted, and that were implanted into one or more surrogate hosts.

The terms "progeny" and "progeny of the transgenic animal" refer to any and all offspring of every generation subsequent to the originally transformed mammals.

The term "mammal" refers to all members of the class Mammalia, including humans, higher primates, domestic and farm animals, such as rabbit, pig, sheep, goat, cattle, and zoo, sports, or pet animals, and rodents, such as mouse and rat.

The term "non-human mammal" refers to all members of the class Mammalia except humans.

As used herein, the expressions "cell," "cell line," and "cell culture" are used interchangeably and all such designations include progeny. Thus, the words "transformants" and "transformed cells" include the primary subject cell and cultures derived therefrom without regard for the number of transfers. It is also understood that all progeny may not be precisely identical in DNA content, due to deliberate or inadvertent mutations. Mutant progeny that have the same function or biological activity as screened for in the originally transformed cell are included. Where distinct designations are intended, it will be clear from the context.

"Tumor", as used herein, refers to all neoplastic cell growth and proliferation, whether malignant or benign, and all pre-cancerous and cancerous cells and tissues.

The terms "cancer" and "cancerous" refer to or describe the physiological condition in mammals that is typically characterized by unregulated cell growth. Examples of cancer include, but are not limited to, carcinoma, lymphoma, blastoma, sarcoma, and leukemia or lymphoid malignancies. More particular examples of such cancers include squamous cell cancer (e.g. epithelial squamous cell cancer), lung cancer including small-cell lung cancer, non-small cell lung cancer, adenocarcinoma of the lung and squamous carcinoma of the lung, cancer of the peritoneum, hepatocellular cancer, gastric or stomach cancer including gastrointestinal cancer, pancreatic cancer, glioblastoma, cervical cancer, ovarian cancer, liver cancer, bladder cancer, hepatoma, breast cancer, colon cancer, rectal cancer, colorectal cancer, endometrial or uterine carcinoma, salivary gland carcinoma, kidney or renal cancer, prostate cancer, vulval cancer, thyroid cancer, hepatic carcinoma, anal carcinoma, penile carcinoma, as well as head and neck cancer.

The term "cytotoxic agent" as used herein refers to a substance that inhibits or prevents the function of cells and/or causes destruction of cells. The term is intended to include radioactive isotopes (e.g. At²¹¹, I¹³¹, I¹²⁵, Y⁹⁰, Re¹⁸⁶, Re¹⁸⁸, Sm¹⁵³, Bi²¹², P³² and radioactive isotopes of Lu), chemotherapeutic agents, and toxins such as small molecule toxins or enzymatically active toxins of bacterial, fungal, plant or animal origin, including fragments and/or variants thereof.

A "chemotherapeutic agent" is a chemical compound useful in the treatment of cancer. Examples of chemotherapeutic agents include alkylating agents such as thiotepa and cyclosphosphamide (CYTOXAN®); alkyl sulfonates such as busulfan, improsulfan and piposulfan; aziridines such as benzodopa, carboquone, meturedopa, and uredopa; ethylenimines and methylamelamines including altretamine, triethylenemelamine, trietylenephosphoramide, triethiylenethiophosphoramide and trimethylolomelamine; acetogenins (especially bullatacin and bullatacinone); delta-9-tetrahydrocannabinol (dronabinol, MARINOL®); beta-lapachone; lapachol; colchicines; betulinic acid; a camptothecin (including the synthetic analogue topotecan (HYCAMTIN®), CPT-11 (irinotecan, CAMPTOSAR®), acetylcamptothecin, scopolectin, and 9-aminocamptothecin); bryostatin; callystatin; CC-1065 (including its adozelesin, carzelesin and bizelesin synthetic analogues); podophyllotoxin; podophyllinic acid; teniposide; cryptophycins (particularly cryptophycin 1 and cryptophycin 8); dolastatin; duocarmycin (including the synthetic analogues, KW-2189 and CB1-TM1); eleutherobin; pancratistatin; a sarcodictyin; spongistatin; nitrogen mustards such as chlorambucil, chlornaphazine, cholophosphamide, estramustine, ifosfamide, mechlorethamine, mechlorethamine oxide hydrochloride, melphalan, novembichin, phenesterine, prednimustine, trofosfamide, uracil mustard; nitrosureas such as carmustine, chlorozotocin, fotemustine, lomustine, nimustine, and ranimnustine; antibiotics such as the enediyne antibiotics (e. g., calicheamicin, especially calicheamicin gamma1I and calicheamicin omegaI1 (see, e.g., Agnew, Chem Intl. Ed. Engl., 33:183-186 (1994)); dynemicin, including dynemicin A; an esperamicin; as well as neocarzinostatin chromophore and related chromoprotein enediyne antiobiotic chromophores), aclacinomysins, actinomycin, authramycin, azaserine, bleomycins, cactinomycin, carabicin, carminomycin, carzinophilin, chromomycinis, dactinomycin, daunorubicin, detorubicin, 6-diazo-5-oxo-L-norleucine, doxorubicin (including ADRIAMYCIN®, morpholino-doxorubicin, cyanomorpholino-doxorubicin, 2-pyrrolino-doxorubicin, doxorubicin HCl liposome injection (DOXIL®), liposomal doxorubicin TLC D-99 (MYOCET®), pegylated liposomal doxorubicin (CAELYX®), and deoxydoxorubicin), epirubicin, esorubicin, idarubicin, marcellomycin, mitomycins such as mitomycin C, mycophenolic acid, nogalamycin, olivomycins, peplomycin, potfiromycin, puromycin, quelamycin, rodorubicin, streptonigrin, streptozocin, tubercidin, ubenimex, zinostatin, zorubicin; anti-metabolites such as methotrexate, gemcitabine (GEMZAR®), tegafur (UFTORAL®), capecitabine (XELODA®), an epothilone, and 5-fluorouracil (5-FU); folic acid analogues such as denopterin, methotrexate, pteropterin, trimetrexate; purine analogs such as fludarabine, 6-mercaptopurine, thiamiprine, thioguanine; pyrimidine analogs such as ancitabine, azacitidine, 6-azauridine, carmofur, cytarabine, dideoxyuridine, doxifluridine, enocitabine, floxuridine; anti-adrenals such as aminoglutethimide, mitotane, trilostane; folic acid replenisher such as frolinic acid; aceglatone; aldophosphamide glycoside; aminolevulinic acid; eniluracil; amsacrine; bestrabucil; bisantrene; edatraxate; defofamine; demecolcine; diaziquone; elfornithine; elliptinium acetate; etoglucid; gallium nitrate; hydroxyurea; lentinan; lonidainine; maytansinoids such as maytansine and ansamitocins; mitoguazone; mitoxantrone; mopidanmol; nitraerine; pentostatin; phenamet; pirarubicin; losoxantrone; 2-ethylhydrazide; procarbazine; PSK® polysaccharide complex (JHS Natural Products, Eugene, Oreg.); razoxane; rhizoxin; sizofiran; spirogermanium; tenuazonic acid; triaziquone; 2,2',2"-trichlorotriethylamine; trichothecenes (especially T-2 toxin, verracurin A, roridin A and anguidine); urethan; dacarbazine; mannomustine; mitobronitol; mitolactol; pipobroman; gacytosine; arabinoside ("Ara-C"); thiotepa; taxoid, e.g., paclitaxel (TAXOL®), albumin-engineered nanoparticle formulation of paclitaxel (ABRAXANE®), and docetaxel (TAXOTERE®); chloranbucil; 6-thioguanine; mercaptopurine; methotrexate; platinum agents such as cisplatin, oxaliplatin, and carboplatin; vincas, which prevent tubulin polymerization from forming microtubules, including vinblastine (VELBAN®), vincristine (ONCOVIN®), vindesine (ELDISINE®, FILDESIN®), and vinorelbine (NAVELBINE®); etoposide (VP-16); ifosfamide; mitoxantrone; leucovovin; novantrone; edatrexate; daunomycin; aminopterin; ibandronate; topoisomerase inhibitor RFS 2000; difluorometlhylornithine (DMFO); retinoids such as retinoic acid, including bexarotene (TARGRETIN®); bisphosphonates such as clodronate (for example, BONEFOSO® or OSTAC(R)), etidronate (DIDROCAL®), NE-58095, zoledronic acid/zoledronate (ZOMETA®), alendronate (FOSAMAX®), pamidronate (AREDIA®), tiludronate (SKELID®), or risedronate (ACTONEL®); troxacitabine (a 1,3-dioxolane nucleoside cytosine analog); antisense oligonucleotides, particularly those that inhibit expression of genes in signaling pathways implicated in aberrant cell proliferation, such as, for example, PKC-α, Raf, H-Ras, and epidermal growth factor receptor (EGF-R); vaccines such as THERATOPE® vaccine and gene therapy vaccines, for example, ALLOVECTIN® vaccine, LEUVECTIN® vaccine, and VAXID® vaccine; topoisomerase 1 inhibitor (e.g., LURTOTECAN®); rmRH (e.g., ABARELIX®); BAY439006 (sorafenib; Bayer); SU-11248 (Pfizer); perifosine, COX-2 inhibitor (e.g., celecoxib or etoricoxib), proteosome inhibitor (e.g., PS341); bortezomib (VELCADE®); CCI-779; tipifarnib (R11577); orafenib, ABT510; Bcl-2 inhibitor such as oblimersen sodium (GENASENSE®); pixantrone; EGFR inhibitors (see definition below); tyrosine kinase inhibitors (see definition below); and pharmaceutically acceptable salts, acids or derivatives of any of the above; as well as combinations of two or more of the above such as CHOP, an abbreviation for a combined therapy of cyclophosphamide, doxorubicin, vincristine, and prednisolone, and FOLFOX, an abbreviation for a treatment regimen with oxaliplatin (ELOXATIN®) combined with 5-FU and leucovovin.

The term "prodrug" as used in this application refers to a precursor or derivative form of a pharmaceutically active substance that is less cytotoxic to tumor cells compared to the parent drug and is capable of being enzymatically activated or converted into the more active parent form. See, e.g., Wilman, "Prodrugs in Cancer Chemotherapy" Biochemical Society Transactions, 14, pp. 375-382, 615th Meeting Belfast (1986) and Stella et al., "Prodrugs: A Chemical Approach to Targeted Drug Delivery," Directed Drug Delivery, Borchardt et al., (ed.), pp. 247-267, Humana Press (1985). The prodrugs of this invention include, but are not limited to, phosphate-containing prodrugs, thiophosphate-containing prodrugs, sulfate-containing prodrugs, peptide-containing prodrugs, D-amino acid-modified prodrugs, glycosylated prodrugs, β-lactam-containing prodrugs, optionally substituted phenoxyacetamide-containing prodrugs or optionally substituted phenylacetamide-containing prodrugs, 5-fluorocytosine and other 5-fluorouridine prodrugs which can be converted into the more active cytotoxic free drug. Examples of cytotoxic drugs that can be derivatized into a prodrug form for use in this invention include, but are not limited to, those chemotherapeutic agents described above.

A "liposome" is a small vesicle composed of various types of lipids, phospholipids and/or surfactant which is useful for delivery of a drug (such as the anti-ErbB2 antibodies disclosed herein and, optionally, a chemotherapeutic agent) to a mammal. The components of the liposome are commonly arranged in a bilayer formation, similar to the lipid arrangement of biological membranes.

A "small molecule" is defined herein to have a molecular weight below about 500 Daltons.

The term "antibody" herein is used in the broadest sense and specifically covers intact monoclonal antibodies, polyclonal antibodies, multispecific antibodies (e.g. bispecific antibodies) formed from at least two intact antibodies, and antibody fragments, so long as they exhibit the desired antigen-binding activity.

The term "monoclonal antibody" as used herein refers to an antibody obtained from a population of substantially homogeneous antibodies, i.e., the individual antibodies comprising the population are identical except for possible naturally occurring mutations that may be present in minor amounts. Monoclonal antibodies are highly specific, being directed against a single antigenic site. Furthermore, in contrast to polyclonal antibody preparations which include different antibodies directed against different determinants (epitopes), each monoclonal antibody is directed against a single determinant on the antigen. In addition to their specificity, the monoclonal antibodies are advantageous in that they may be synthesized uncontaminated by other antibodies. The modifier "monoclonal" indicates the character of the antibody as being obtained from a substantially homogeneous population of antibodies, and is not to be construed as requiring production of the antibody by any particular method. For example, the monoclonal antibodies to be used in accordance with the present invention may be made by the hybridoma method first described by Kohler et al., Nature, 256:495 (1975), or may be made by recombinant DNA methods (see, e.g., U.S. Pat. No. 4,816,567). The "monoclonal antibodies" may also be isolated from phage antibody libraries using the techniques described in Clackson et al., Nature, 352:624-628 (1991) and Marks et al., J. Mol. Biol., 222:581-597 (1991), for example.

The monoclonal antibodies herein specifically include "chimeric" antibodies in which a portion of the heavy and/or light chain is identical with or homologous to corresponding sequences in antibodies derived from a particular species or belonging to a particular antibody class or subclass, while the remainder of the chain(s) is identical with or homologous to corresponding sequences in antibodies derived from another species or belonging to another antibody class or subclass, as well as fragments of such antibodies, so long as they exhibit the desired biological activity (U.S. Pat. No. 4,816,567; and Morrison et al., Proc. Natl. Acad. Sci. USA, 81:6851-6855 (1984)). Chimeric antibodies of interest herein include primatized antibodies comprising variable domain antigen-binding sequences derived from a non-human primate (e.g. Old World Monkey, Ape etc) and human constant region sequences.

"Antibody fragments" comprise a portion of an intact antibody, preferably comprising the antigen-binding or variable region thereof. Examples of antibody fragments include Fab, Fab', F(ab')₂, and Fv fragments; diabodies; linear antibodies; single-chain antibody molecules; and multispecific antibodies formed from antibody fragment(s).

An "intact" antibody is one which comprises an antigen-binding variable region as well as a light chain constant domain (C_{L}) and heavy chain constant domains, CHI, CH2 and CH3. The constant domains may be native sequence constant domains (e.g. human native sequence constant domains) or amino acid sequence variant thereof. Preferably, the intact antibody has one or more effector functions.

"Humanized" forms of non-human (e.g., rodent) antibodies are chimeric antibodies that contain minimal sequence derived from non-human immunoglobulin. For the most part, humanized antibodies are human immunoglobulins (recipient antibody) in which residues from a hypervariable region of the recipient are replaced by residues from a hypervariable region of a non-human species (donor antibody) such as mouse, rat, rabbit or nonhuman primate having the desired specificity, affinity, and capacity. In some instances, framework region (FR) residues of the human immunoglobulin are replaced by corresponding non-human residues. Furthermore, humanized antibodies may comprise residues that are not found in the recipient antibody or in the donor antibody. These modifications are made to further refine antibody performance. In general, the humanized antibody will comprise substantially all of at least one, and typically two, variable domains, in which all or substantially all of the hypervariable loops correspond to those of a non-human immunoglobulin and all or substantially all of the FRs are those of a human immunoglobulin sequence. The humanized antibody optionally also will comprise at least a portion of an immunoglobulin constant region (Fc), typically that of a human immunoglobulin. For further details, see Jones et al., Nature 321:522-525 (1986); Riechmann et al., Nature 332:323-329 (1988); and Presta, Curr. Op. Struct. Biol. 2:593-596 (1992).

### II. Detailed Description

### Generation of conditional PIK3CA^{H1047R} non-human animal models of cancer

As discussed above, the class IA PI3K catalytic subunits, p110α, p110β and p110ó, occur as an obligate heterodimer in complex with p85α, p55α, p50α, p85β or p55γ regulatory subunits. In normal cells, PI3Ks are maintained in a basal inactive state and become active following growth factor stimulation and cell surface receptor engagement. Activated PI3Ks phosphorylate and convert phosphatidylinositol 4, 5 bisphosphate (PIP2) leading to phosphatidylinositol 3, 4, 5 trisphosphate (PIP3). Elevated cellular PIP3 levels promote activation of PI3K-effectors which in-turn regulate a number of cellular processes, including cell growth, proliferation and survival.

Oncogenic mutations in *PIK3CA,* which encodes the phosphoinositide 3-kinase (PI3K) catalytic subunit p110α, occur in over 25% of human breast cancers. Frequent mutations in the *PIK3CA* gene have also been reported in other types of human cancer, such as ovarian, colorectal, gastric, lung, hepatocellular, thyroid and endometrial cancers, acute leukemia and malignancies of the central nervous system.

The present invention is based, at least in part, on the development of a knock-in mouse model for breast cancer where the endogenous *PIK3CA* allele was modified to allow for tissue specific conditional expression of a frequently found *PIK3CA* H1047R (*PIK3CA^{e20H1047R}*) mutant allele. In particular, the endogenous PIK3CA allele was modified by placing a dormant copy of an oncogenic mutant PIK3CA exon, including H1047R, adjacent to the wild-type coding exon. Prior to activation of the dormant mutant allele, the engineered mice expressed the modified wild-type PIK3CA (*PIK3CA^{e20mwt}*)*.* However, upon Cre-mediated recombination the mutant PIK3CA H1047R allele (*PIK3CAe^{20H1047R}*) was activated, leading to its expression. The results presented herein show that the conditional activation of PIK3CA H1047R allele in mouse mammary epithelium leads to mutant PIK3CAH1047R expression and tumorigenesis. Further, in performing a whole exome analysis of spindle cell tumors from this model, the emergence of a spontaneous loss-of-function TP53 mutation was identified as a potential cooperating event involved in tumor formation.

It has been found that the activation of the latent *PIK3CA* H1047R allele resulted in breast tumors with multiple histological types. Consistent with the expression of *PIK3CA^{e20H1047R},* all tumors showed activation of the PI3K pathway. Further, whole exome analysis of the *PIK3CA* H1047R driven mammary tumors identified multiple mutations including a well characterized TP53 hotspot mutation that appeared spontaneously during the development of spindle cell type tumors. Apart from demonstrating the utility of the model in studying the emergence of secondary mutations during tumorigenesis, the efficacy of GDC-0941, a PI3K-inhibitor in clinical development, was tested using this mouse model and it was shown that the tumors responded to PI3K inhibition. Accordingly, the transgenic non-human animal model developed herein is a valuable tool to study tumor initiation, progression and treatment, and also lends itself to identification of lesions that cooperate with mutant PIK3CA during tumor development. In particular, the non-human transgenic animal model of the present invention finds utility in the screening of anti-cancer agents, and the *PIK3CA^{e20H1047R}* and the secondary mutations, alone or in any combination, find utility in the diagnosis, classification, prognosis, and treatment of cancer, including, but not limited to, breast cancer.

In generating the transgenic mice of the present invention, the Cre-lox recombination system was used. The Cre protein is a site-specific DNA recombinase, which can catalyze the recombination of DNA between specific sites in a DNA molecule. These sites, known as *loxP* sequences, contain specific binding sites for Cre that surround a directional core sequence where recombination can occur. When cells that have *loxP* sites in their genome express Cre, a recombination event can occur between the *loxP* sites. The double stranded DNA is cut at both *loxP* sites by the Cre protein. The strands are then rejoined with DNA ligase. The result of recombination depends on the orientation of the *loxP* sites. For two lox sites on the same chromosome arm, inverted *loxP* sites will cause an insertion, while a direct repeat of *loxP* sites will cause a deletion event.

While the Cre-lox recombination system was initially developed for use in activating gene expression in mammalian cell lines and transgenic mice, it has since shown that Cre-Lox recombination can be used to delete loxP-flanked chromosomal DNA sequences at high efficiency in various selected cell types of transgenic animals, and that the Cre-lox recombination can be used for conditional gene targeting in vivo. In addition, other site-directed recombination systems, including those listed before, can be used to generate non-human transgenic animal models similar to the mouse model specifically exemplified herein.

Due to the importance of PIK3CA mutations in breast cancer, the role of the PIK3CA H1047R allele was tested in breast tumorigenesis by breeding a *PIK3CA^{e20mwt}* mouse to a MMTV-Cre transgenic mouse, where the Cre recombinase was expressed under the control of the mammary gland permissive MMTV LTR promoter. For other types of tumors other tissue permissive or tissue specific promoters can be used. Tissue specific promoters, including regulatory elements resulting in expression of specific tissue types or organs, such as liver, pancreas, kidney, liver, lung, testis, are known in the art and are available, for example, from the TiProD database of human promoter sequences for which some functional features are known. The database allows users to query individual promoters and the expression pattern they mediate, and to retrieve sets of promoters according to their tissue-specific activity. Non-human transgenic animal models of other cancer types where PIK3CA mutations play a role can be generated, for example, by using one of the appropriate tissue specific promoters known in the art.

The transgenic non-human animals of the present invention, including their progeny, can be screened for the presence and/or expression of the transgene by any suitable method. Screening is often accomplished by Southern blot or PCR of DNA prepared from tail tissue, using a probe that is complementary to at least a portion of the transgene. Western blot analysis or immunohistochemistry using an antibody against the protein encoded by the transgene may be employed as an alternative or additional method for screening for the presence of the transgene product. Alternatively, the tissues or cells believed to express the transgene at the highest levels are tested for the RNA expression of the transgene using Northern analysis or RT-PCR.

Alternative or additional methods for evaluating the presence of the transgene include, without limitation, suitable biochemical assays such as enzyme and/or immunological assays, histological stains for particular marker or enzyme activities, flow cytometric analysis, and the like. Analysis of the blood may also be useful to detect the presence of the transgene product in the blood, as well as to evaluate the effect of the transgene on the levels of various types of blood cells and other blood constituents.

### Screening of Candidate Anti-Cancer Agents

The non-human transgenic animal models of the present invention find utility in screening candidate agents for anti-cancer activity. The term "anti-cancer activity" is used in the broadest sense and includes activity in directly or indirectly mediating any effect in preventing, delaying, slowing down or inhibiting tumor development and/or growth which may provide for a beneficial effect to the host. "Anti-cancer activity" thus encompasses, without limitation, the ability to, directly or indirectly, reduce the number of cancer cells, reduce the tumor size or tumor load, inhibit (i.e., slow to some extent and preferably stop) cancer cell infiltration into peripheral organs, inhibit (i.e., slow to some extent and preferably stop) tumor metastasis, inhibit, to some extent, tumor growth, and/or relieve to some extent one or more of the symptoms associated with the cancer.

Agents having anti-cancer activity that finds clinical use in treatment of cancer are of particular interest. Of particular interest in the present disclosure is the identification of agents that have an anti-cancer activity that can provide a clinical benefit in treating cancer characterized by oncogenic mutations in PIK3CA, and in particular by activation of the mutant PIK3CA H1047R allele or a secondary mutation.

In general, anti-cancer activity is assessed by evaluating the presence or absence of an effect upon a cancer phenotype in the non-human transgenic animal model, which effect on cancer phenotype is indicative of the anti-cancer activity of the candidate agent. Thus, for example, a reduction in tumor burden in the animal following administration of a candidate agent indicates the agent has anti-cancer activity.

The candidate anti-cancer agents that can be screened for anti-cancer activity using the non-human animal model of the present invention include, without limitation, synthetic, naturally occurring, or recombinantly produced molecules, including small molecules, peptides, antibodies and other polypeptides,
Candidate agents can be obtained from a wide variety of sources including libraries of synthetic or natural compounds. For example, numerous means are available for random and directed synthesis of a wide variety of organic compounds and biomolecules, including expression of randomized oligonucleotides and oligopeptides. Alternatively, libraries of natural compounds in the form of bacterial, fungal, plant and animal extracts are available or readily produced. Additionally, natural or synthetically produced libraries and compounds are readily modified through conventional chemical, physical and biochemical means, and may be used to produce combinatorial libraries. Known pharmacological agents may be subjected to directed or random chemical modifications, such as acylation, alkylation, esterification, amidification, etc. to produce structural analogs.

The candidate agent can be administered in any manner desired and/or appropriate for delivery of the agent in order to examine anti-cancer activity. For example, the candidate agent can be administered by injection (e.g., by injection intravenously, intramuscularly, subcutaneously, and the like), infusion, orally, or by any other desirable means.

The screening method can involve administering varying amounts of the candidate agent (from no agent to an amount of agent that approaches an upper limit of the amount that can be delivered successfully to the animal, e.g., within toxicity limits), and may include delivery of the agent in different formulations and routes. The agents can be administered singly or can be combined in combinations of two or more, especially where administration of a combination of agents may result in a synergistic effect.

The ability of a candidate agent to treat a preexisting cancer can be assessed by administering the candidate agent to the transgenic animal, and evaluating modulation of a cancer phenotype. Modulation of a cancer phenotype can be assessed, for example, by evaluating the presence or absence of an effect on, for example, tumor burden, number of tumors, tumor size, metabolic activity of tumor cells, progression-free survival (PFS), overall survival (OFS), and the like.

The ability of a candidate agent to facilitate prevention of cancer can be assessed by administering the candidate agent prior to induction of mutation resulting in the development of tumor. Prevention of cancer refers to reduction in the incidence and/or severity of tumors in the animal relative to the incidence and/or severity of cancer expected in the absence of intervention (e.g., without administration of an agent having anti-cancer activity).

Further details of the invention are illustrated by the following non-limiting example.

### Example

### Conditionally Activatable PIK3CAH1047R Mouse Model

### Materials and Methods

### Generation of a conditionally activatable PIK3CA H1047R mice

We used a targeting vector template (**Fig. 1**) to assemble the final targeting vector. Using mouse genomic DNA from C57B/6N ES cells as template, a 2.045kb fragment encompassing *PIK3CA* exons 18-19 (left arm), a 588bp region containing *PIK3CA* wild-type exon 20 (mid arm), and a 3.137kb genomic DNA also containing exon 20 and additional 3' intronic sequence (left arm) was PCR amplified and used for constructing the vector. Prior to cloning into the template targeting vector codon 1047 encoded by exon 20 in the left arm was mutated to code for histidine (R1047H) using standard mutagenesis techniques. In cloning the mid-arm into the targeting template vector, the wild-type exon 20 region was modified by the addition of a high polyA signal present in the vector. A neomycin expression cassette, a 4x transcriptional stop cassette (Jackson, E.L. et al. Genes Dev 15, 3243-8 (2001)) was also provided by the vector. In the final configuration, the neomycin cassette was flanked by 'frt' sites to facilitate its excision following modification of the ES cells. Also, the modified wild-type exon was flanked by 'loxP' sites to facilitate its removal, upon which the mutant exon 20 can be activated (**Fig. 1**). The accuracy of the targeting vector was verified by Sanger sequencing. The targeting construct was electroporated into C57B/6N ES cells and selected for neomycin resistance. Appropriately targeted ES clones were identified by 5' and 3' Southern blotting. The 5' probe recognized a 6.6 kb wild-type fragment and a 9.1 kb fragment when appropriately targeted genomic DNA was tested following AflII digestion and southern blotting. Similarly, the 3' probe recognized a 6.9 kb wild-type fragment and a 4.8 kb fragment in the appropriately modified genomic region following SacI digestion (**Fig 1c**). Following removal of the neo cassette and confirmation of the architecture of the modified genomic region encoding PIK3CA using PCR and sequencing, the ES clones were injected into blastocysts to generate chimeric mice. A 290 bp PCR product, as opposed to a 201bp fragment in the wild-type mice, generated using primer 20F (AGCCAGCAGACAATAATTCTTAGCACA) (SEQ ID NO: 1) and 20R (CAGTGCTTGAACATTGGAGGTCAGT) (SEQ ID NO: 2) was used to follow germline transmission and also genotype modified *PIK3CA* allele bearing mice. To activate the latent *PIK3CA H1047R* mutant allele in mammary glands, we bred this mouse with an MMTV-Cre (Jackson labs # 003551) line. For out end point study, mice with palpable tumors were followed until tumor reached -2500 mm³ and were then euthanized. Also, mice that exhibited any body condition score <2 (Ullman-Cullere, M.H. & Foltz, C.J. B Lab Anim Sci 49, 319-23 (1999), hunched posture and >20% loss of body weight, were also euthanized. All mice were maintained in our animal facility in accordance with Institutional Animal Care and Use Committee (IACUC) guidelines.

### DNA/RNA preparation

DNA from tumors/tissues was prepared using Qiagen AllPrep DNA/RNA kit (Qiagen, CA). RNA samples were prepared using RNeasy Mini Kit (Qiagen, CA).

### Expression analysis

Quantity and quality of total RNA samples was determined using ND-1000 spectrophotometer (Thermo Scientific, DE) and Bioanalyzer 2100 (Agilent Technologies, CA), respectively. Cy-dye labeled cRNA and array hybridization was prepared as manufacturer's (Agilent Technologies, CA) instructions. Briefly, total RNA sample was converted to double-stranded cDNA and then to Cy-dye labeled cRNA using Agilent's Quick Amp Labeling Kit. The labeled cRNA was purified using RNeasy mini kit (Qiagen, CA). cRNA yield and Cy-dye incorporation was determined using ND-1000 spectrophotometer (Thermo Scientific, Wilmington, DE). 750 ng of the labeled cRNA was fragmented and hybridized to the Agilent's Whole Mouse Genome 4x44K arrays. All samples were labeled with Cy5-dye and hybridized against Cy3-dye labeled universal mouse reference (Stratagene, CA). Samples were hybridized for 17 hours at 65°C with a constant agitation on Pro Hybridization station HS 4800 (Tecan US, NC). Following hybridization, the arrays were washed, dried and scanned on Agilent scanner. Agilent's Feature Extraction software 10.7 was used to analyze acquired array images.

Gene expression data were obtained from two-color Agilent microarrays used in a common-reference design experiment. The common reference sample was measured in the Cy3 channel and the sample of interest was measured in the Cy5 channel. The data was processed using methods implemented in the limma R package (Smyth, G.K. Stat Appl Genet Mol Biol 3, Article3 (2004)). First the data were background corrected after which a within-array loess fit normalization, and a between-array quantile normalization were applied (Smyth, G.K. & Speed, T. Methods 31, 265-73 (2003)). Differentially expressed gene signatures were obtained using limma's linear model fit and probes with FDR adjusted p-value of 0.05 or lower and a log₂ change of at least 2 were deemed differentially expressed. Hierarchical clustering of the samples and EMT related genes was computed on the mean centered gene expression profiles using the euclidian distance metric and complete linkage clustering.

### Comparative Genomic Hybridization (CGH) arrays

Mammary tumor samples from *PIK3CA^{e20H1047R}* mice were profiled on Agilent CGH arrays according to the manufacturer's protocol (Agilent Technologies, CA). Briefly, 500 ng DNA of both tumor and a reference sample (C57BL/6J mouse, The Jackson Laboratory, ME) were digested with Alu I and Rsa I (Promega, WI) and subsequently purified with QIAprep Spin Miniprep kit (QIAGEN GmbH, Germany). Digested samples were labeled with Cy5-dUTP (test sample) or Cy3-dUTP (reference sample) using the Genomic DNA Labeling Kit Plus (Agilent Technologies, CA). Test samples were pooled with the reference and subsequently purified using MicroCon YM-30 (Millipore, Billerica, MA). Labeled probes were mixed with Cot-1 DNA (Invitrogen,CA), 10x blocking agent and 2x Hi-RPM hybridization buffer (Agilent Technologies, CA) and hybridized to Agilent's 244K Mouse Genome CGH microarray. The samples were hybridized for 24 hours at 67°C with a constant agitation on Pro Hybridization station HS 4800 (Tecan US, NC). Arrays were washed, dried and scanned on the Agilent scanner according to the manufacturer's protocol (Agilent Technologies, CA). Individual log₂ ratios of background subtracted signal intensities were obtained from the Agilent Feature Extraction software version 10.7. The log₂ ratios were centered to a median of zero and the resulting log₂ ratio values for each probe were segmented using GLAD (Hupe, P., et al. Bioinformatics 20, 3413-22 (2004)). All genes within the genomic bounds of a given GLAD-derived segment were given the mean copy number value of the probes within that segment. Copy number values >= 0.4 log₂ ratio units represented gain and values <= -0.4 log₂ ratio units represented loss.

### Exome capture and sequencing

Targeted sequence capture was performed using 3 µg of genomic DNA and SureSelectXT Mouse All Exon kit according to manufacturer's protocol (Agilent Technologies, CA). The Mouse All Exon kit is designed to capture 49.6 Mb of targeted region and covers 221,784 exons within 24,306 genes. Pre-capture library was amplified using four PCR cycles whereas 12 PCR cycles were used in post-capture amplification. Fragment size distribution of post-capture amplified libraries was determined on Bioanalyzer 2100 using DNA high sensitivity chip (Agilent Technologies, CA). Concentration of the libraries was measured by Kapa library quantification kit (Kapa Biosystems, MA). Each library was sequenced on HiSeq 2000 to generate 2 x 75 bp reads per manufacturer instructions (Illumina, CA). The sequencing reads were mapped to UCSC mouse genome (NCBI37/mm9) using BWA software (Li, H. & Durbin, R. Bioinformatics 25, 1754-60 (2009)) set to default parameters. Local realignment, duplicate marking and raw variant calling were performed as described previously (DePristo, M.A. et al. Nature Genetics 43, 491-498 (2011)). SNPs represented in dbSNP Build 131 (Sherry, S.T. et al. Nucleic Acids Res 29, 308-11 (2001)) was used to remove known germline variations. In addition variants that were present in both the tumor and normal samples were removed as germline variations. Remaining variations present in the tumor sample, but absent in the matched normal were predicted to be somatic. Predicted somatic variations were additionally filtered to include only positions with a minimum of 10x coverage in both the tumor and matched normal as well as an observed variant allele frequency of <1% in the matched normal. The effect of protein somatic mutation on gene function was predicted using SIFT (Ng, P.C. & Henikoff, S. Genome Res 12, 436-46 (2002)) and PolyPhen (Ramensky, V., Bork, P. & Sunyaev, S. H, Nucleic Acids Res 30, 3894-900 (2002)) (**Table 1**).

### Histological, immunohistochemical and whole mount analysis

Five micron, formalin-fixed, paraffin-embedded specimens was used for routine Hematoxylin and Eosin (H&E) staining and histology evaluation.

For whole mount analysis the mammary glands were dissected, spread and fixed with Carnoy's fixative for 2-4 hours. The tissues were hydrated and stained in Carmine alum overnight.

Immunoflurescence staining was performed using 10 micron sections Tissue-Tek OCT (Sakura Finetek, CA) embedded frozen samples. Sectioned samples were fixed in 4% paraformadehyde for 10 min and then blocked for 30min with PBT (PBS with 0.1% Triton) containing 1% BSA. The blocked sections were then stained with appropriate primary antibody diluted in PBT with 0.1% BSA overnight at 4° C in a humidified chamber. The slides were washed 3 times in PBT and then incubated with appropriate secondary antibodies for 60 min at room temperature in a humidified chamber. Unbound secondary antibody was removed by washing with PBT. Prolong Gold anti-fade reagent (Invitrogen, CA) was used to mount the slides. Primary antibodies used in the study are cytokeratin 5 (Abcam, ab53121), cytokeratin18 (Abcam, ab668), ERα (Thermo scientific, Ab-21), PR (Abcam, ab2764) and Vimentin (Abcam, ab45939). Appropriate secondary antibodies conjugated with Alexa 488 or 647 (Molecular probes) were used for detecting the bound primary antibody.

### Western blot analysis

Frozen tumors were weighed and lysed with a pestle PP (Scienceware, NJ) in cell extract buffer (Invitrogen, CA) supplemented with protease inhibitors (F. Hoffman-La Roche, Ltd., Germany), ImM phenylmethysufonyl fluoride, and phosphatase inhibitor cocktails 1 and 2 (Sigma, MO). Protein concentrations were determined using the Pierce BCA Protein Assay Kit (Rockford, IL). For Western blots equal amounts of protein were separated by electrophoresis through NuPage Bis-Tris 4-12% gradient gels (Invitrogen, CA), proteins were transferred onto nitrocellulose pore membranes using the iBlot system (Invitrogen, CA). Primary antibodies used for blotting include pAkt (Ser473), pAkt (Thr308), total Akt, pS6 (Ser235/236), pS6 (Ser240/242) and total S6 antibodies were obtained from Cell Signaling Technology (Danvers, MA), and anti-actin antibody was purchased from Sigma-Aldrich (St. Louis, MO). Chemiluminescence western blotting detection (Amersham Biosciences, PA) coupled with appropriate secondary horseradish peroxidase-conjugated secondary IgG antibodies was employed, to detect the primary antibody bound proteins.

### Drug efficacy study

Mammary tumor pieces were implanted subcutaneously near mammary fat pad of 8-10 week female C.B-17 SCID beige mice (Charles River Laboratories, MA) weighing between 20-25g. Tumors were monitored until they reached a mean tumor volume of 200-300 mm³, and animals were randomly distributed into 4 groups of 9 animals each before the initiation of dosing. GDC-0941 was dissolved in 0.5% methylcellulose with 0.2% Tween 80 (MCT) vehicle and dosed daily for 13 days by oral gavage in three dosing groups: 50, 100 and 150 mg/kg. Tumor sizes and mouse body weights were recorded twice weekly over the course of the study. Tumor volumes were measured in two dimensions with a caliper and calculated with the following formula: Tumor size (mm3) = (longer measurement × shorter measurement2) × 0.5. Percent weight change was calculated using the following formula: Group percent weight change = (new weight - initial weight)/ initial weight) × 100. Mean tumor volumes (+/- SEM) and percent weight change were calculated and plotted using Kaleidagraph (Version 4.03, Synergy Software). Mice with tumor volumes ≥2,000 mm³ or with losses in body weight of ≥20% from their initial body weight were promptly euthanized based on IACUC guideline. To analyze the repeated measurement of tumor volumes from the same animals over time, a mixed modeling approach was used (Pinheiro, J., Bates, D., DebRoy, S., Sarkar, D. & The R Core team. nlme: Linear and Nonlinear Mixed Effects Models. R package version 3.1-89. (2008)). This approach addresses both repeated measurements and modest dropouts due to any non-treatment related death of animals before study end. Cubic regression splines were used to fit a non-linear profile to the time courses of log₂ tumor volume at each dose level. These non-linear profiles were then related to dose within the mixed model. Tumor growth inhibition as a percentage of vehicle (%TGI) was calculated as the percentage of the area under the fitted curve (AUC) for the respective dose group per day in relation to the vehicle, using the formula: %TGI = 100 × (1 - AUCdose/AUCveh). To get uncertainty intervals (UIs) for %TGI, the fitted curve and the fitted covariance matrix were used to generate a random sample as an approximation to the distribution of %TGI. The random sample is composed of 1000 simulated realizations of the fitted-mixed model, where the %TGI has been recalculated for each realization. Our reported UI is the values for which 95% of the time, the recalculated values of %TGI will fall in this region given the fitted model. The 2.5 and 97.5 percentiles of the simulated distribution were used as the upper and lower UIs. Plots were generated using R (version 2.8.1, R Development Core Team 2008; R Foundation for Statistical Computing; Vienna, Austria) and Excel. Data was analyzed using R and the mixed models were fitted within R using the nlme package (Pinheiro et al., supra).

### Results

### Engineering conditionally activatable PIK3CA H1047R mice

To study the role of *PIK3CA* mutation in tumor initiation, development and progression we have engineered a mouse capable of conditionally expressing mutant *PIK3CA* H1047R allele driven by its native promoter. The engineered mouse, *PIK3CAe^{20mwt},* has a wild-type *PIK3CA* exon 20 that was modified to contain flanking loxP sites. The modified wild-type exon is followed by a transcriptional stop cassette and a copy of *PIK3CA* exon 20 encoding the H1047R mutation (**Fig. 1a****-d**). A targeting vector (**Fig. 1b**) was used to modify the endogenous *PIK3CA* locus in mouse embryonic stem (ES) cells. Two independent ES cell clones containing the appropriate modification, identified by Southern blotting (**Fig. 1f****, g**), were used to generate chimeric mice that showed germline transmission of the *PIK3CA^{e20mwt}* allele. Intercrosses involving heterozygous *PIK3CA*^{*e20mwt*/+} mice resulted in progenies with the appropriate genotypes at the expected Mendelian ratios (**Fig. 13**). Unlike the embryonic lethality observed in *PIK3CA*^{*-*/*-*} null mice (Bi, L., Okabe, et al., J Biol Chem 274, 10963-8 (1999)), prior to Cre-mediated recombination, we found that the homozygous *PIK3CA*^{*e20mw*/*le20mwt*} animals were born at expected Mendelian frequency, indicating that the modified *PIK3CA^{e20mwt}* functioned analogous to the wild-type *PIK3CA* allele.

### Mammary gland specific expression of PIK3CA^{e20H1047R} allele

Since *PIK3CA* is mutated in over 25% of human breast cancers (Zhao & Voigt, Oncogene 27, 5486-5496 (2008)), we tested the role of the *PIK3CA* H1047R in breast tumorigenesis by breeding the *PIK3CA^{e20mwt}* mouse to a MMTV-Cre transgenic mouse (Wagner, K.U. et al. Nucleic Acids Res 25, 4323-30 (1997); Wagner, K.U. et al. Transgenic Res 10, 545-53 (2001)). The MMTV-Cre strain expresses PI Cre recombinase under the control of the mammary gland permissive MMTV LTR promoter, allowing for recombination and expression of *PIK3CA^{e20H1047R} .* We confirmed the expression of the *PIK3CA^{e20H1047R}* mutant allele and the native wild-type *PIK3CA* allele by sequencing cDNA corresponding to the *PIK3CA* mRNA extracted from mammary glands of the *PIK3CA*^{*e20H1047R*/+} mouse (**Fig. 1h**). In contrast, RNA extracted from kidney showed only the expression of the wild-type *PIK3CA* from the *PIK3CA^{e20mwt}* allele (**Fig. 1i**), confirming the mammary-specific expression of the mutant *PIK3CA^{e20H1047R}* allele.

### Expression of PIK3CA^{20H1047R} leads to enhanced mammary branch morphogenesis

Previous studies have shown that expression of oncogenes or loss of tumor suppressors, like PTEN, affect branching and morphogenesis of mouse mammary glands (Meyer, D.S. et al. Cancer Research (2011); Li, G. et al., Development 129, 4159-70 (2002); Fishler, T. et al., Oncogene 29, 4007-17 (2010). Hence, we studied the effect of expression of *PIK3CA^{e20H1047R}* on mammary gland development using whole-mount staining. At 12 weeks, *PIK3CA^{e20H1047R}* mutant mammary gland showed hyper-branched ductal and terminal structures compared to the normal terminal end buds (TEBs) in control mammary gland (**Fig. 6a****, b**). By 50 weeks, the *PIK3CA^{e20H1047R}* mutant mammary glands showed feathery hyper-branched morphology compared to control mammary glands (**Fig. 6c****, d**). Further, histological sections of the *PIK3CA^{e20H1047R}* mutant mammary glands showed evidence of tumor nodules at 50 weeks of age (**Fig 6e****, f**). This is similar to the mammary branching morphogenesis defects reported in previous studies involving PTEN conditional null mice and other mammary specific transgenic models with PI3K pathway activation (Meyer et al., supra; Li et al., supra).

### PIK3CA^{e20H1047R} expression promotes mammary tumorigenesis

Female *PIK3CA*^{*e20H1047R*/+}, *PIK3CA*^{*e20mwt*/*+*} and MMTV-Cre mice were followed for mammary tumors development over an 80 week period. We found that the heterozygous *PIK3CA*^{*e20H1047R*/+} animals developed mammary gland tumors (**Fig. 2a****-c**) with a median tumor free survival of 478.5 days. Majority of the animals developed tumors in the thoracic mammary glands (# 1, 2, 3, 6 7 & 8), while some developed tumors in the inguino-abdominal mammary glands (# 4, 5, 9 & 10), and a few developed tumors in both the thoracic and inguino-abdominal mammary glands. In contrast to the *PIK3CA*^{*e20H1047R*/+}*,* the control *PIK3CA*^{*e20mwt*/+} and MMTV-Cre animals remained tumor free for the duration of the study. The mammary gland tumor phenotype with a similar latency was also observed in *PIK3CA*^{*e20H1047R*/+} mice derived from a second independent targeted ES clone (**Fig. 2c**), further confirming the specificity of the *PIK3CA^{e20H1047R}* expression driven tumor phenotype.

The MMTV promoter drives the expression of genes under its control in both virgin and lactating mammary glands (Wagner, K.U. et al. Nucleic Acids Res 25, 4323-30 (2010). Given this, we compared the tumor incidence in nulliparous and multiparous mice and found that the latency was significantly shortened in the multiparous *PIK3CA^{e20H1047R}* mice compared to nulliparous *PIK3CA^{e20H1047R}* animals (**Fig. 7**) from 492 to 465 days (p=0.0002), suggesting that pregnancy related mammary gland changes contribute to accelerated tumorigenesis.

While MMTV promoter directs the expression of Cre starting at day six after birth primarily in mammary epithelial cells, it is known to also drive expression in salivary glands and male seminal vesicles (Wagner, K.U. et al. Transgenic Res 10, 545-53 (2001)). In performing histological analysis of salivary glands from mice bearing mammary tumors and seminal vesicles from males aged between 35-41 weeks, we found no evidence of tumors (**Fig. 8a****, b**). However, morphologically the seminal vesicles were larger in *PIK3CA*^{*e20H1047R*/+} mice (n=3) compared to the control animals. Histological analysis of seminal vesicle cross sections indicated an increase in the amount of seminal vesicle fluid secretion leading to ductal distention and enlargement (**Fig. 8c****, d**). However, this did not affect the breeding and fertility of *PIK3CA*^{*e20H1047R*/+} males.

### Histology and signaling activation in PIK3CA^{e20H1047R} driven tumors

We performed histological analysis of *PIK3CA^{e20H1047R}* tumors to understand its pathological features and found that the majority of the tumors showed features consistent with fibroadenoma (76.9% [20/26]) (**Fig. 2d**), while a few showed histopathological features of adenocarcinoma (15.4% [4/26]) (**Fig. 2e****, f**) or spindle cell neoplasia (7.7% [2/26]) (**Fig. 2g**).

To further characterize the tumors we tested the PI3K pathway activation status of the histologically distinct tumor types. In all tumors types we observed elevated pAKT and pS6-kinase (**Fig. 2h**), both downstream of PI3K, indicative of constitutive activation of the pathway in these tumors.

In an effort to further understand the mammary tumor types observed, we studied the expression of basal cytokeratin 5 (CK5), luminal cytokeratin 18 (CK18), estrogen receptor (ER), progesterone receptor (PR), and vimentin, a mesenchymal marker, in the histologically distinct mammary tumor types. While we found that the fibroadenomas and adenocarcinomas were both positive for CK5, CK18, ER and PR, the spindle cell tumors were ER⁻/PR⁻ (**Fig. 3n****, o**) and CK5⁻ (data not shown). Interestingly, this is reminiscent of human mammary spindle cell tumors that are also ER/PR negative. In addition, the spindle cell tumors were CK18⁺/vimentin⁺(**Fig. 3n****, p**), indicating that the cells are undergoing epithelial-mesenchymal transition (EMT).

### Genomic analysis of PIK3CA^{e20H-1047R} driven mammary tumors

Prior to performing genomic analysis of the *PIK3CA* H1047R mammary tumors, given the long latency for tumor formation, we first tested if the tumor explants derived from the mammary tumors would grow as xenografts in SCID mice. A majority of the fibroadenomas, adenocarcinomas and the spindle cell tumors that were tested formed tumors. Interestingly, the histology of tumors derived from fibroadenomas explants showed features of adenocarcinoma or spindle cell neoplasia, indicating that that fibroadenoma are likely benign and that only a subset of the cells within the original explants with tumorigenic potential contributed to the development of tumors upon passage (**Fig. 9a****-d**). However, the spindle cell tumor explants maintained its histological features during passage (**Fig. 9e****, f**).

We performed microarray-based gene expression analyses of the different mammary tumor subtypes and control mammary glands to further understand the molecular characteristics of the tumors. Hierarchical clustering of the expression data using markers for epithelial cells, stem cells and EMT (Weigelt, B. & Reis-Filho, Nature Reviews Clinical Oncology 6, 718-730 (2009); Taube, J.H. et al. Proceedings of the National Academy of Sciences 107, 15449-15454 (2010)) showed that primary fibroadenomas, adenocarcinomas and the tumors derived from passaging explants from these tumor types clustered together indicating a common origin and shared set of gene expression changes (**Fig. 4a**). In contrast the spindle cell type, both primary and passaged tumors, clustered together displaying features of EMT, suggesting a distinct cellular origin (**Fig. 4a**).

Whole exome capture and sequencing has been applied in identifying somatic mutations in coding regions of genomes (Varela, I. et al. Nature 469, 539-542 (2011)). In order to understand the emergence of secondary mutations in the various mammary tumor types observed in our model we performed whole exome capture and sequencing of a selected set of samples (**Fig. 14****.** Besides the presence of the engineered *PIK3CA* H1047R mutation, the tumors contained several additional somatic changes (**Table 1**). We found that the fibroadenomas had a low level of somatic mutations (between 2 and 5), followed by adenocarcinoma (22 mutations) and spindle cell tumors (61 mutations). Notably, in the spindle cell tumor we identified a mutation in Trp53 (*TP53*) at codon 245 that replaced the Arg with His (**Fig. 4b**). This mutation is the equivalent of human *TP53* hotspot mutation at codon 248 (R248H) and is a well characterized loss-of-function mutation within the DNA-binding domain of TP53 that renders it to act as dominant negative protein (Olivier, M., Hollstein, M. & Hainaut, P. Cold Spring Harbor Perspectives in Biology 2, a001008-a001008 (2010)). Further the *TP53* mutation and majority of the mutations identified in the primary spindle cell tumor were also found in the explant-derived tumors following passage indicating that the primary spindle tumor was likely less heterogeneous (**Fig. 10**). As observed in the spindle cell tumors, majority of the mutations present in the primary adenomcarinoma were present in the tumor derived following transplantation (**Fig. 10**). In adenocarcinoma, of the 22 mutations observed, 12 including mutations in Plk1, Tssk2, and SMG1 kinase are predicted to have a functional effect and hence might act as drivers (**Table 1 &** **Fig. 10**). Interestingly, SMG1 mutations in human breast cancer have been previously reported (Stephens, P. et al., Nature Genetics 37, 590-592 (2005).

In addition to the whole exome mutational analysis, we profiled the tumors on CGH arrays to understand chromosomal copy number alteration. Interestingly, mirroring the low level of somatic mutations observed in fibroadenomas and adenocarcinomas, both these tumor types had very few regions of copy number aberrations. In contrast to fibroadenomas and adenocarcinomas, in spindle cell tumor samples we found several regions with copy number alterations when compared to normal mammary glands. We observed a broad amplification in chromosome 11 in a ∼3Mb region and focal gains in chromosome 1 and 4. For further details, see, Yuan W. et al., Oncogene (2012), 1-9, including the Supplementary Information accompanying the paper on the Oncogene website (http://www.nature.com/onc). Spindle cell tumors are also characterized by two regions of chromosomal loss that fall within chromosome 11 and 12, both of which contain several coding genes. In particular, deletion in chromosome 11 resulted in loss of *Nf1* (**Fig. 4C**) and is consistent with its down regulated expression (log₂ ratio of -2.09; p = 8.338e-05, **Fig 4A**), suggesting that the Ras-MAPK pathway might also be engaged in these tumors.

### PIK3CA^{20H-1047R} mammary tumors respond to PI3K inhibitor

Genetically engineered mouse models provide an ideal platform for candidate drug testing in a preclinical setting (Singh, M. et al. Nat Biotechnol 28, 585-93 (2010)). Since the mice in this study develop mammary tumors after a long latency, we used our tumor explant-derived tumor model to test its utility in assessing drug efficacy. Explants derived from spindle cell tumor with *TP53* mutations were implanted subcutaneously near the mammary fat pad and treated with a PI3KCA inhibitor, GDC-0941 (Edgar, K.A. et al. Cancer Research 70, 1164-1172 (2010); Folkes, A. et al. WO 2007127175), once the tumors had reached at least ∼ 200mm³ . Tumor bearing mice were treated in 4 groups of 9 animals each with 50, 100 and 150 mg/kg of GDC-0941 or vehicle. We found that the animals treated with GDC-0941 showed the highest tumor growth inhibition (TGI) of 85% at 150mg/kg, although tumor growth inhibition was also observed at the other doses tested compared to vehicle treatment (**Fig. 5a**), indicating that these tumors were still dependent on the mutant PIK3CA signaling for their growth. Further, log-rank tests of this data on time to progression showed that all the drug treatment groups showed a statistically significant difference when compared to vehicle control treated animals at all the doses tested (p <0.0001 at 150mg/kg; **Fig. 12**). Consistent with the TGI observed, tumor from mice treated with GDC-0941 showed a significant decrease in pAKT and pS6 at least up to 10 hours following treatment (**Fig 5b**), confirming the contribution of PI3K inhibition in tumor growth inhibition.

### Discussion

Genetically engineered mouse models of cancer are invaluable for understanding tumor initiation, progression and therapy evaluation (Tuveson, D. & Hanahan, D. Nature 471, 316-7 (2011)). In addition, application of next generation sequencing technologies to these models can provide valuable information on the molecular aberrations that arise during the evolution of tumors. Here we describe a conditionally activatable *PIK3CA* H1047R mouse model of breast cancer and show that these mice develop mammary tumors of multiple histological types that include fibroadenomas, adenocarcinomas and spindle cell neoplasia. We found that the adenocarcinomas were positive for hormone receptors and cytokeratins (CK5⁺/CK18⁺) indicating that they are epithelial in origin. In contrast, the spindle cell tumors, while positive for cytokeratin 18, were distinct in that they were ER/PR negative, but vimentin positive. This is reminiscent of human mammary spindle tumors (Khan, H. European Journal of Surgical Oncology 29, 600-603 (2003); Carter, M.R., et al. Am J Surg Pathol 30, 300-9 (2006)). The multiple histological types observed in our breast cancer model is consistent with previous studies involving transgenic models of *PIK3CA* H1047R driven breast cancer in mouse (Adams, J.R. et al. Cancer Research 71, 2706-2717 (2011); Meyer, D.S. et al. Cancer Research (2011)). However, the latency for breast tumor development in our model is longer compared to the two studies where the *PIK3CA* H1047R mutant cDNA was expressed under the control of either a ROSA26 locus promoter (median survival 150 days in line A; >500 days in line NLST) or chicken β-actin promoter (average 214 ± 22.6 days) following activation of its expression using MMT-Cre. This likely reflects the fact that our knock-in model expresses the mutant *PIK3CA* allele from its endogenous promoter present from its normal genomic configuration and hence mimics more accurately *PIK3CA* H1047R driven tumorigenesis *in vivo.*

The advent of next generation sequencing technology has allowed comprehensive characterization of cancers at the sequence level (Stratton, M.R., et al., Nature 458, 719-724 (2009)). In applying next generation sequencing technology we have characterized tumors derived from our mouse model to understand the additional hits required for breast tumor development following expression of *PIK3CA H1047R* mutant allele. Among several candidate secondary hits that may cooperate with *PIK3CA^{H1047R}* we report the occurrence of a *TP53^{R245H}* mutation in the spindle cell tumors. The R245H mutation is the equivalent of R248H hotspot dominant-negative mutant observed in human cancers. The spontaneous appearance of the loss-of-function *TP53^{R245H}* mutation, the fact that *TP53* mutations co-occur with *PIK3CA* mutations in breast cancer (Boyault, S. et al. Breast Cancer Research and Treatment (2011)) and that loss of *TP53* has been shown to cooperate with oncogenic *PIK3CA* in a transgenic mouse model (Adams, J.R. et al. Cancer Research 71, 2706-2717 (2011)) suggests that this is likely a cooperating driver mutation. This indicates that our model mimics the lesions that contribute to human mammary tumorigenesis and hence would be an ideal model to study therapeutic intervention in mutant *PIK3CA* driven mammary tumors. In line with this, we demonstrate the utility of the model in testing the efficacy of GDC-0941, a PI3K inhibitor, in a subset of the mammary tumor with EMT features. A previous report studied the efficacy of NVP-BEZ235, a dual pan-PI3K and mammalian target of rapamycin (mTOR) inhibitor, in a *PIK3CA* H1047R transgenic lung tumor model. However, given that the *PIK3CA* mutations are more frequent in human breast cancers, our model can serve as a genetically defined system to test efficacy of PI3K inhibitors in breast cancer and potentially help model treatment regimens for clinical drug testing.

Besides its utility in studying mammary tumorgenesis, the model can be used to study other cancers where *PIK3CA* mutations have been observed by activating the mutant *PIK3CA* allele using an appropriate tissue specific Cre line. Further the model in conjunction with next generation sequencing technologies can be used to discover and understand other driver genes that cooperate with mutant *PIK3CA* in additional tumor types. Given that PI3K pathway activation has been implicated in resistance to therapy, the *PI3KCA* H1047R mice can also be used to model drug resistance and study therapeutic strategies to overcome resistance.

## Claims

1. A non-human transgenic mammal, which is a rodent, comprising a modified endogenous *PIK3CA* locus, wherein said modified endogenous *PIK3CA* locus comprises a dormant mutant *PIK3CA* exon 20 allele encoding a H1047R mutation downstream to wild-type exon 20 followed by a transcriptional stop cassette, wherein said wild-type exon 20 followed by a transcriptional stop cassette is flanked by loxP sites, and wherein said non-human transgenic animal is capable of conditional expression of the *PIK3CA* H1047R (*PIK3CA^{e20H1047R}*) mutant allele, wherein the conditional expression of said mutant allele is under control of the *PIK3CA* endogenous promoter and wherein said conditional expression is triggered by activation of said mutant allele by Cre-mediated recombination.

2. The non-human transgenic mammal of claim 1, wherein said conditional expression leads to mutant PIK3CAH1047R expression and tumorigenesis.

3. A tumor-bearing non-human transgenic mammal, which is a rodent, conditionally expressing a mutant *PIK3CA* allele encoding a H1047R (*PIK3CA^{e20H1047R}*) mutation under control of the *PIK3CA* endogenous promoter.

4. The non-human transgenic mammal of claim 1 or the tumor-bearing non-human transgenic mammal of claim 3 expressing the *PIK3CA* H1047R mutant allele in a tissue-specific manner.

5. The non-human transgenic mammal or the tumor-bearing non-human transgenic mammal of claim 4 wherein the *PIK3CA* H1047R mutant allele is expressed in the mammary gland of said animal and the tumor is a mammary tumor.

6. The tumor-bearing non-human transgenic mammal of claim 5, which is heterozygous for the *PIK3CA* H1047R mutant allele.

7. The tumor-bearing non-human transgenic mammal of claim 5, which is homozygous for the *PIK3CA* H1047R mutant allele.

8. The tumor-bearing non-human transgenic mammal of claim 5, wherein the mammary tumor is selected from the group consisting of fibroadenomas, adenocarcinomas and spindle cell neoplasia.

9. The non-human transgenic mammal of claim 1 or the tumor-bearing non-human transgenic mammal of claim 3, which is a mouse or a rat.

10. A method for making a transgenic mammal of claim 1, comprising placing a copy of exon 20 encoding a H1047R mutation, downstream to exon 20 encoding the wild-type *PIK3CA* allele followed by a transcriptional stop cassette, wherein said exon 20 encoding the wild-type *PIK3CA* allele followed by a transcriptional stop cassette is flanked by loxP sites.

11. A method of screening a drug candidate for the treatment of tumor comprising (a) administering a drug candidate selected from a small molecule, peptide, polypeptide, or antibody, to the tumor-bearing non-human mammal of claim 3, and (b) measuring the response of the tumor to said treatment, wherein step (b) optionally comprises evaluating the ability of said drug candidate to evoke at least one response selected from the group consisting of reduction of the number of tumor cells, reduction of tumor size or tumor load, inhibition of tumor cell infiltration into peripheral organs, inhibition of tumor metastasis, and inhibition of tumor growth.

12. The method of claim 11, wherein the tumor is selected from the group consisting of fibroadenomas, adenocarcinomas and spindle cell neoplasias.

13. The method of claim 11, wherein the tumor is selected from the group consisting of breast cancer, ovarian cancer, colorectal cancer, gastric cancer, lung cancer, hepatocellular cancer, thyroid cancer, endometrial cancer, leukemia and malignancies of the central nervous system.

14. A method for identifying an anti-cancer agent for the treatment of drug-resistant cancer, comprising (a) administering a drug candidate to the tumor-bearing non-human mammal of claim 3, (b) measuring the response of the tumor to said treatment, and (c) identifying said drug candidate as an anti-cancer agent for the treatment of drug-resistant cancer if the tumor exhibits a positive response to said treatment, wherein optionally said positive response is selected from the group consisting of reduction of the number of tumor cells, reduction of tumor size or tumor load, inhibition of tumor cell infiltration into peripheral organs, inhibition of tumor metastasis, and inhibition of tumor growth.

15. A method for identifying a PI3K inhibitor, comprising (a) administering a PI3K inhibitor candidate to the tumor-bearing non-human mammal of claim 3, (b) measuring the response of the tumor to said treatment, and (c) identifying said candidate as a PI3K inhibitor if the tumor exhibits a positive response to said treatment.

16. The method of claim 15, wherein said cancer is breast cancer.

17. Use of the tumor-bearing non-human mammal of claim 3 in a method of screening a drug candidate for the treatment of tumor comprising (a) administering a drug candidate to the tumor-bearing non-human animal of claim 3, and (b) measuring the response of the tumor to said treatment.

## Patentansprüche

1. Nicht-menschliches, transgenes Säugetier, das ein Nagetier ist, umfassend einen modifizierten endogenen PIK3CA-Locus, wobei der modifizierte endogene *PIK3CA*-Locus ein ruhendes mutiertes PIK3CA-Exon-20-Allel, das für eine H1047R-Mutation, stromabwärts des Wildtyp-Exons 20, codiert, gefolgt von einer Transkriptionsstoppkassette umfasst, wobei das Wildtyp-Exon 20, gefolgt von einer Transkriptionsstoppkassette, von loxP-Stellen flankiert wird, und wobei das nicht-menschliche transgene Tier zur konditionalen Expression des PIK3CA-H1047R-Mutantenallels (*PIK3CA^{e20H1047R}*-Mutantenallels) fähig ist, wobei die konditionale Expression des mutierten Allels unter Kontrolle des endogenen *PIK3CA-*Promotors steht und wobei die konditionale Expression durch Aktivierung des mutierten Allels durch Cre-vermittelte Rekombination ausgelöst wird.

2. Nicht-menschliches, transgenes Säugetier nach Anspruch 1, wobei die konditionale Expression zu mutierter PIK3CAH1047R-Expression und Tumorgenese führt.

3. Tumortragendes, nicht-menschliches, transgenes Säugetier, das ein Nagetier ist, das ein mutiertes *PIK3CA*-Allel, das für eine H1047R-Mutation (*PIK3CA^{e20H1047R}*-Mutation) codiert, unter Kontrolle des endogenen PIK3CA-Promotors konditional exprimiert.

4. Nicht-menschliches, transgenes Säugetier nach Anspruch 1 oder tumortragendes, nicht-menschliches, transgenes Säugetier nach Anspruch 3, das das *PIK3CA*-H1047R-Mutantenallel in einer gewebespezifischen Weise exprimiert.

5. Nicht-menschliches, transgenes Säugetier oder tumortragendes, nicht-menschliches, transgenes Säugetier nach Anspruch 4, wobei das *PIK3CA*-H1047R-Mutantenallel in der Brustdrüse des Tieres exprimiert wird und der Tumor ein Brusttumor ist.

6. Tumortragendes, nicht-menschliches, transgenes Säugetier nach Anspruch 5, das für das *PIK3CA*-H1047R-Mutantenallel heterozygot ist.

7. Tumortragendes, nicht-menschliches, transgenes Säugetier nach Anspruch 5, das für das *PIK3CA*-H1047R-Mutantenallel homozygot ist.

8. Tumortragendes, nicht-menschliches, transgenes Säugetier nach Anspruch 5, wobei der Brusttumor aus der Gruppe bestehend aus Fibroadenomen, Adenokarzinomen und Spindelzellneoplasien ausgewählt ist.

9. Nicht-menschliches, transgenes Säugetier nach Anspruch 1 oder tumortragendes, nicht-menschliches, transgenes Säugetier nach Anspruch 3, das eine Maus oder eine Ratte ist.

10. Verfahren zum Herstellen eines transgenen Säugetiers nach Anspruch 1, umfassend das Platzieren einer Kopie von Exon 20, das für eine H1047R-Mutation codiert, stromabwärts des Exons 20, das für das Wildtyp-*PIK3CA*-Allel codiert, gefolgt von einer Transkriptionsstoppkassette, wobei das Exon 20, das für das Wildtyp-*PIK3CA*-Allel codiert, gefolgt von einer Transkriptionsstoppkassette, von loxP-Stellen flankiert wird.

11. Verfahren zum Screenen eines Arzneistoffkandidaten zur Tumorbehandlung, umfassend (a) Verabreichen eines Arzneistoffkandidaten, ausgewählt aus einem kleinen Molekül, einem Peptid, einem Polypeptid oder einem Antikörper, an das tumortragende nicht-menschliche Säugetier nach Anspruch 3, und (b) Messen des Ansprechens des Tumors auf die Behandlung, wobei Schritt (b) gegebenenfalls das Bewerten der Fähigkeit des Arzneistoffkandidaten, mindestens ein Ansprechen auszulösen, das aus der Gruppe bestehend aus Verringerung der Anzahl an Tumorzellen, Verringerung der Tumorgröße oder Tumorlast, Hemmung der Tumorzelleninfiltration in periphere Organe, Hemmung der Tumormetastasierung und Hemmung des Tumorwachstums ausgewählt ist, umfasst.

12. Verfahren nach Anspruch 11, wobei der Tumor aus der Gruppe bestehend aus Fibroadenomen, Adenokarzinomen und Spindelzellneoplasien ausgewählt ist.

13. Verfahren nach Anspruch 11, wobei der Tumor aus der Gruppe bestehend aus Brustkrebs, Eierstockkrebs, Dickdarmkrebs, Magenkrebs, Lungenkrebs, hepatozellulärem Krebs, Schilddrüsenkrebs, Endometriumkarzinom, Leukämie und Malignitäten des zentralen Nervensystems ausgewählt ist.

14. Verfahren zum Identifizieren eines Antikrebsmittels zur Behandlung von arzneistoffresistentem Krebs, umfassend (a) Verabreichen eines Arzneistoffkandidaten an das tumortragende Säugetier nach Anspruch 3, (b) Messen des Ansprechens des Tumors auf die Behandlung und (c) Identifizieren des Arzneistoffkandidaten als Antikrebsmittel zur Behandlung von arzneistoffresistentem Krebs, wenn der Tumor ein positives Ansprechen auf die Behandlung zeigt, wobei gegebenenfalls das positive Ansprechen aus der Gruppe bestehend aus einer Verringerung der Anzahl an Tumorzellen, Verringerung der Tumorgröße oder Tumorlast, Hemmung der Tumorzelleninfiltration in periphere Organe, Hemmung der Tumormetastasierung und Hemmung des Tumorwachstums ausgewählt ist.

15. Verfahren zum Identifizieren eines PI3K-Hemmers, umfassend (a) Verabreichen eines PI3K-Hemmer-Kandidaten an das tumortragende, nicht-menschliche Säugetier nach Anspruch 3, (b) Messen des Ansprechens des Tumors auf die Behandlung und (c) Identifizieren des Kandidaten als einen PI3K-Hemmer, wenn der Tumor ein positives Ansprechen auf die Behandlung zeigt.

16. Verfahren nach Anspruch 15, wobei der Krebs Brustkrebs ist.

17. Verwendung des tumortragenden, nicht-menschlichen Säugetiers nach Anspruch 3 in einem Verfahren zum Screenen eines Arzneistoffkandidaten zur Tumorbehandlung, umfassend (a) Verabreichen eines Arzneistoffkandidaten an das tumortragende, nicht-menschliche Tier nach Anspruch 3 und (b) Messen des Ansprechens des Tumors auf die Behandlung.

## Revendications

1. Mammifère transgénique non humain, qui est un rongeur, comprenant un locus *PIK3CA* endogène modifié, dans lequel ledit locus *PIK3CA* endogène modifié comprend un allèle mutant dormant d'exon 20 de *PIK3CA* codant pour une mutation H1047R en aval de l'exon 20 de type sauvage suivi d'une cassette d'arrêt de transcription, dans lequel ledit exon 20 de type sauvage suivi d'une cassette d'arrêt de transcription est bordé de sites loxP, et dans lequel ledit animal transgénique non humain est capable d'expression conditionnelle de l'allèle mutant *PIK3CA* H1047R (*PIK3CA^{e20H1047R}*)*,* dans lequel l'expression conditionnelle dudit allèle mutant est sous le contrôle du promoteur endogène de *PIK3CA* et dans lequel ladite expression conditionnelle est déclenchée par activation dudit allèle mutant par une recombinaison induite par Cre.

2. Mammifère transgénique non humain selon la revendication 1, dans lequel ladite expression conditionnelle conduit à l'expression de PIK3CAH1047R mutant et à une tumorigenèse.

3. Mammifère transgénique non humain porteur de tumeur, qui est un rongeur, exprimant conditionnellement un allèle mutant de *PIK3CA* codant pour une mutation H1047R (*PIK3CA^{e20H1047R}*) sous le contrôle du promoteur endogène de *PIK3CA.*

4. Mammifère transgénique non humain selon la revendication 1 ou mammifère transgénique non humain porteur de tumeur selon la revendication 3, exprimant l'allèle mutant *PIK3CA* H1047R d'une façon tissu-spécifique.

5. Mammifère transgénique non humain ou mammifère transgénique non humain porteur de tumeur selon la revendication 4, dans lequel l'allèle mutant *PIK3CA* H1047R est exprimé dans la glande mammaire dudit animal et la tumeur est une tumeur mammaire.

6. Mammifère transgénique non humain porteur de tumeur selon la revendication 5, qui est hétérozygote pour l'allèle mutant *PIK3CA* H1047R.

7. Mammifère transgénique non humain porteur de tumeur selon la revendication 5, qui est homozygote pour l'allèle mutant *PIK3CA* H1047R.

8. Mammifère transgénique non humain porteur de tumeur selon la revendication 5, dans lequel la tumeur mammaire est choisie dans le groupe constitué par les fibroadénomes, les adénocarcinomes et une néoplasie à cellules fusiformes.

9. Mammifère transgénique non humain selon la revendication 1 ou mammifère transgénique non humain porteur de tumeur selon la revendication 3, qui est une souris ou un rat.

10. Procédé de préparation d'un mammifère transgénique selon la revendication 1, comprenant la mise en place d'une copie de l'exon 20 codant pour une mutation H1047R, en aval de l'exon 20 codant pour l'allèle de *PIK3CA* de type sauvage suivi d'une cassette d'arrêt de transcription, dans lequel ledit exon 20 codant pour l'allèle de *PIK3CA* de type sauvage suivi d'une cassette d'arrêt de transcription est bordé de sites loxP.

11. Procédé de criblage d'un médicament candidat pour le traitement d'une tumeur comprenant (a) l'administration d'un médicament candidat choisi parmi une petite molécule, un peptide, un polypeptide ou un anticorps, au mammifère non humain porteur de tumeur selon la revendication 3, et (b) la mesure de la réponse de la tumeur audit traitement, dans lequel l'étape (b) comprend éventuellement l'évaluation de l'aptitude dudit médicament candidat à susciter au moins une réponse choisie dans le groupe constitué par une réduction du nombre de cellules tumorales, une réduction de la taille de la tumeur ou de la charge tumorale, une inhibition de l'infiltration de cellules tumorales dans des organes périphériques, une inhibition des métastases tumorales, et une inhibition de la croissance tumorale.

12. Procédé selon la revendication 11, dans lequel la tumeur est choisie dans le groupe constitué par les fibroadénomes, les adénocarcinomes et les néoplasies à cellules fusiformes.

13. Procédé selon la revendication 11, dans lequel la tumeur est choisie dans le groupe constitué par un cancer du sein, un cancer de l'ovaire, un cancer colorectal, un cancer gastrique, un cancer du poumon, un cancer hépatocellulaire, un cancer de la thyroïde, un cancer de l'endomètre, une leucémie et des malignités du système nerveux central.

14. Procédé d'identification d'un agent anticancéreux pour le traitement d'un cancer résistant aux médicaments, comprenant (a) l'administration d'un médicament candidat au mammifère non humain porteur de tumeur selon la revendication 3, (b) la mesure de la réponse de la tumeur audit traitement, et (c) l'identification dudit médicament candidat en tant qu'un agent anticancéreux pour le traitement d'un cancer résistant aux médicaments si la tumeur manifeste une réponse positive audit traitement, dans lequel éventuellement ladite réponse positive est choisie dans le groupe constitué par une réduction du nombre de cellules tumorales, une réduction de la taille de la tumeur ou de la charge tumorale, une inhibition de l'infiltration de cellules tumorales dans des organes périphériques, une inhibition des métastases tumorales, et une inhibition de la croissance tumorale.

15. Procédé d'identification d'un inhibiteur de PI3K, comprenant (a) l'administration d'un candidat inhibiteur de PI3K au mammifère non humain porteur de tumeur selon la revendication 3, (b) la mesure de la réponse de la tumeur audit traitement, et (c) l'identification dudit candidat en tant qu'inhibiteur de PI3K si la tumeur manifeste une réponse positive audit traitement.

16. Procédé selon la revendication 15, dans lequel ledit cancer est un cancer du sein.

17. Utilisation du mammifère non humain porteur de tumeur selon la revendication 3 dans un procédé de criblage d'un médicament candidat pour le traitement d'une tumeur, comprenant (a) l'administration d'un médicament candidat à l'animal non humain porteur de tumeur selon la revendication 3, et (b) la mesure de la réponse de la tumeur audit traitement.
